(19) Europäisches Patentamt
European Patent Office
Office européen des brevets

(11) **EP 4 353 147 A2**

(12) **EUROPEAN PATENT APPLICATION**

(43) Date of publication:
**17.04.2024 Bulletin 2024/16**

(21) Application number: **24155478.1**

(22) Date of filing: **23.11.2021**

(51) International Patent Classification (IPC):
**A61B 5/0215** (2006.01)

(52) Cooperative Patent Classification (CPC):
**A61B 5/07; A61B 5/0031; A61B 5/062;
A61B 5/076; F16C 32/0408;** A61B 5/0215;
A61B 5/686; A61B 5/6861; A61B 5/6869;
F16C 2316/10

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.12.2020 EP 20211518**

(62) Document number(s) of the earlier application(s) in
accordance with Art. 76 EPC:
**21814788.2 / 4 255 285**

(71) Applicant: **Koninklijke Philips N.V.
5656 AG Eindhoven (NL)**

(72) Inventors:
• **GLEICH, Bernhard
Eindhoven (NL)**
• **RAHMER, Jürgen Erwin
Eindhoven (NL)**

(74) Representative: **Philips Intellectual Property &
Standards
High Tech Campus 52
5656 AG Eindhoven (NL)**

Remarks:
This application was filed on 02.02.2024 as a
divisional application to the application mentioned
under INID code 62.

(54) **MICRODEVICE FOR ALLOWING A LOCALIZATION OF THE MICRODEVICE**

(57)     The invention relates to a medical microdevice 100 for insertion into a human body, wherein the microdevice allows for measuring at least one of a localization of the microdevice in a space and/or a physical parameter in the environment of the microdevice, wherein the microdevice comprises a casing 111 and within the casing a magneto mechanical rotator 110, wherein the magneto mechanical rotator comprises a magnetic object 113 providing a permanent magnetic moment and a rotary bearing 112 that is adapted to stabilize a rotational motion of the magnetic object, wherein the magneto mechanical rotator is adapted to transduce an external magnetic or electromagnetic excitation field into a mechanical rotation of the magnetic object relative to the rotary bearing such that a periodically changing magnetic response field is generated. The microdevice thus allows for an improved signal transmission and for a further miniaturization.

FIG. 1

**Description**

FIELD OF THE INVENTION

The invention relates to a medical microdevice allowing for a localization of the medical microdevice in space, and a reading system for reading out a signal from the microdevice. Further, the invention relates to a method for localizing the medical microdevice and for determining a physical parameter in an environment of the medical microdevice, and to a computer program product for localizing the medical microdevice and/or for determining the physical parameter.

BACKGROUND OF THE INVENTION

Recently, very small mechanical devices have been developed, for instance, in the form of microbots, that can advantageously be utilized in applications that introduce strict size constraints, for instance, medical applications within the human body. Such microdevices are in particular useful in the form of localization or sensor devices. Although such microdevices already exist with an approximate size below one millimeter, in localization and sensor applications these microdevices often suffer from a low signal strength that does not allow for a further miniaturization of these devices. It would thus be desirable to provide microdevices that provide for an improved signal transmission and allow for a further miniaturization.

WO 2019/243098 A1 discloses a microdevice comprising a magneto mechanical oscillator utilizing a suspension wire attached to a magnetic object. Utilizing a suspension wire is not only very expensive with a low production yield, due to the complex manufacturing steps necessary for attaching the suspension wire to the magnetic object, but also prevents a further miniaturization of the microdevice. In particular, the size of such a microdevice depends on the availability of suspension wire diameters, and thus the decreasing of a size of the microdevices strongly depends on the development of suspension wires with smaller diameters. Hence it would be desirable to provide a microdevice with a much simpler design allowing for a further miniaturization, a high production yield and a less expensive production. Moreover, the signal provided by microdevice described in document WO 2019/243098 A1 is limited to the resonance frequency that can be achieved by the magneto mechanical oscillator, wherein this frequency depends on the size and magnetic moment of the magnetic objects of the magneto mechanical oscillator and is in reality limited to below 10 kHz. However, it would be desirable to provide microdevices that can provide a signal with a higher signal frequency, for instance, 50 kHz, which imply a higher signal to noise ratio and a higher measurement frequency allowing, for instance, an application as a pressure sensor in a heart implant. Compared with the microdevices of WO 2019/243098 A1 it is thus desirable to provide an improved microdevice that provides a signal with an improved signal-to-noise ratio and is at the same time easier to manufacture while still allowing for a further miniaturization.

SUMMARY OF THE INVENTION

It is therefore an object of the present invention to provide an improved microdevice for localization and/or sensor purposes that allows, in particular, for an improved signal transmission and for a further miniaturization. It is further an object to provide an improved microdevice that allows for an easier and less costly manufacturing. The invention is defined by the independent claims. The dependent claims define advantageous embodiments.

According to a first aspect of the invention, a medical microdevice for insertion into a human body is presented, wherein the microdevice allows for measuring at least one of a location of the microdevice in space and/or a physical parameter in the environment of the microdevice, wherein the microdevice comprises a casing and within the casing a magneto mechanical rotator, wherein the magneto mechanical rotator comprises a magnetic object providing a permanent magnetic moment and a rotary bearing that is adapted to stabilize a rotational motion of the magnetic object, wherein the magneto mechanical rotator is adapted to transduce an external magnetic or electromagnetic excitation field into a mechanical rotation of the magnetic object relative to the rotary bearing such that a periodically changing response magnetic field is generated.

Since the magneto mechanical rotator comprises a magnetic object that can rotate relative to a rotary bearing such that a response magnetic field is generated, the rotary bearing allows for a stabilization of the magnetic object and thus for a fast and stable rotation after the excitation of the magneto mechanical rotator. Such a stable rotation generates magnetic signals with higher frequency and higher signal strengths that can be used for localizing the microdevice or for carrying information encoded in the signal. Thus, the microdevice allows for an improved signal transmission and for a further miniaturization.

Generally, microdevices refer to devices that are smaller than 1 millimeter in at least one spatial direction. Preferably, the medical microdevice is smaller than 1 millimeter in at least two orthogonal spatial directions. Further preferred, the medical microdevice is smaller than 1 millimeter in all spatial directions, i.e. in each spatial direction. Preferably, the medical microdevice comprises a size that allows it to be introduced into a circulatory system of a human being. However, even smaller sizes are possible for the medical microdevice. The physical parameter that is measured by the medical mircodevice refers preferably to a pressure in the environment of the medical microdevice. However, the physical parameter can also refer to any other physical parameter, for instance, to a temperature.

The casing can be any casing surrounding the magneto mechanical rotator and can be adapted in accordance with a specific application of the microdevice. Preferably, the casing comprises a coating referring preferably to a biocompatible coating. Moreover, if the medical microdevice is utilized as a sensor for sensing a physical parameter in the environment of the medical microdevice, the casing can be adapted such that it allows for such a sensing operation. For instance, if the medical microdevice shall be utilized as pressure sensor, the casing preferably comprises a flexible part that allows to convey external pressure changes to the inside of the casing. If temperature changes in the environment of the medical microdevice shall be measured, the casing can be adapted to easily convey such temperature changes into an inner part of the casing.

The magnetic object provides a permanent magnetic moment and is thus preferably made of a hard magnetic material allowing to keep a once introduced magnetic moment. Preferably, the magnetic object refers to a neodymium magnet with a high remanence larger than N52, i.e. larger than 1.42 T. In applications in environments with a temperature of more than 80°C it is preferred that the magnetic object refers to a neodymium magnet with H, SH, EH or AH characteristics according to the general neodymium magnet identification system. Also CoSm (cobalt samarium) magnets can be employed as magnetic object.

Preferably, the magnetic object comprises a spherical shape which is advantageous for improving a reliability of the medical microdevice. However, in another preferred embodiment, the magnetic object can comprise an oblate spheroidal shape which allows for a higher rotational stability but can be less reliable due to its asymmetric interaction with the rotary bearing. Alternatively, the magnetic object can also comprise a prolate spheroidal shape. Generally, it is preferred that the magnetic object comprises a spheroidal shape which has the highest momentum of inertia in a plane perpendicular to the rotation axis of the magnetic object, i.e. in a direction or along an axis within a plane of rotation of the magnetic object. The rotary bearing is generally adapted to stabilize a position and rotational movement of the rotating magnetic object. The rotary bearing can refer to any mechanical or magnetic rotary bearing that allows for a rotation of the magnetic object and does not provide a mechanical restriction to the rotational movement of the rotating magnetic object. Moreover, the rotary bearing does not refer to a mechanical attachment assembly attaching the rotating magnetic object to the casing or a part of the casing. Thus, attachment means like strings or bellows cannot be regarded as rotary bearing.

The magneto mechanical rotator is adapted to transduce an external magnetic or electromagnetic excitation field into a mechanical rotation of the magnetic object relative to the rotary bearing. In particular, the external magnetic or electromagnetic excitation field refers to an oscillating field comprising a constant or chirped frequency for exciting the rotational motion of the magnetic object within the casing. The rotational motion excited by the external magnetic or electromagnetic excitation field can refer to a complete rotational motion, i.e. to a repeated rotation through 360° in the same direction, or to a rotational oscillation in which the magnetic object rotates repeatedly less than 360° in one direction and afterwards again less than 360° in the opposite direction.

In an embodiment with a completely rotating magnetic object, it is preferred that the magneto mechanical rotator is adapted such that the magnetic object can rotate with a rotation frequency of more than 5 kHz, more preferably 50 kHz, maximal rotation frequency when applying a magnetic excitation field of 1 mT/$\mu_0$, wherein the maximal rotation frequency is a function of the applied field strength. For medical applications within or near a human being the field strength of the excitation field is preferably limited to 4 mT/$\mu_0$ to avoid cellular stimulations. It has been found that the maximum rotation frequency scales with a power between 0.1 and 0.5 of the applied field strength depending on the dampening influences acting on the magnetic object.

If the magnetic object is adapted to perform a rotational oscillation, it is preferred that the magneto mechanical rotator is adapted to allow for an oscillation frequency higher than 500 Hz*mm, preferably higher than 8000 Hz*mm, even more preferably up to 50000 Hz*mm. In this case, the oscillation frequency depends on the size of the magnetic object such that for a magnetic object with a 1 mm radius the oscillation frequency is, for instance, at least 500 Hz, whereas for a 0.1 mm magnetic object the oscillation frequency is, for instance, at least 5 kHz. Generally, it has been found that a practical limit for the oscillation frequency of the magnetic object in the magneto mechanical rotator refers to an oscillation frequency of 50000 Hz*mm.

The magneto mechanical rotator, in particular, the magnetic object and the rotary bearing, are adapted such that following the excitation of the magnetic object the magnetic object rotates relative to the rotary bearing such that a periodically changing response magnetic field is generated by the magnetic object. The generated response magnetic field allows preferably for a localization of the medical microdevice. In particular, the generated response magnetic field of the magnetic object can be measured, for instance, based on an electrical current and/or voltage induced by the generated magnetic field in external magnetic coils, to localize the medical microdevice. In particular, more than one magnetic coil can be used for measuring the response magnetic field of the magnetic object at different positions, wherein based on the different measurements, in particular, based on the different signal strengths, the medical microdevice can be located very accurately.

In an embodiment, the rotary bearing comprises a retaining magnetic field generator adapted for generating a retaining magnetic field such that it provides a saddle point, wherein the magnetic object is positioned substantially at the saddle point, and wherein the saddle point is defined such that, for a spatial plane predefined with respect to the rotary bearing, when the magnetic object is moved away from the saddle point within the spatial plane, the magnetic object is subjected to a magnetic restoring force provided by the retaining magnetic field forcing the magnetic object back into a direction to the saddle point. Thus, the saddle point provides a stable position for a magnetic object in at least two dimensions. Providing the retaining magnetic field such that it provides a saddle point at which the magnetic object can be positioned allows for a stable rotation of the magnetic object while minimizing a physical contact between the magnetic object and the rotary bearing. In this context, positioning the magnetic object substantially at the saddle point means that the magnetic center of the magnetic object is positioned substantially at the saddle point, wherein due to external influences the magnetic object is not all the time positioned directly at the saddle point, but somewhere near the saddle point, wherein the restoring forces forcing the magnetic object back to the saddle point become the greater the farther the magnetic object deviates from the saddle point. Preferably, substantially at the saddle point means that the magnetic object is positioned at the saddle point such that a distance of a magnetic center of the magnetic object to the saddle point is less than a dimension of the magnetic object. In some of the embodiments, the magnetic field generator can also be adapted to generate more than one saddle point, wherein then the magnetic object can be positioned in one of these saddle points or additional magnetic objects can be provided that are positioned at the other provided saddle points.

Generally, an arrangement of the retaining magnetic field generator that provides a retaining magnetic field with a saddle point as described above can be found by utilizing known numerical simulation algorithms that can simulate the magnetic field and/or the magnetic energy that is caused by an arrangement of one or more magnetic objects. For example, such a numerical simulation can generally utilize the following algorithm. First, a possible arrangement of a retaining magnetic field generator is defined, for instance, by the location of permanent or soft magnetic objects forming the retaining magnetic field generator. Then, one or more magnetic objects are placed in relation to the retaining soft magnetic field generator and the overall magnetic energy of the system is calculated using the known physical laws. In a next step, the one or more magnetic objects are displaced infinitesimally in an arbitrary direction and the overall magnetic energy is again calculated for the new arrangement. This last step can then be repeated until an energy landscape for the positions of the magnetic objects is determined, wherein the energy landscape allows for determining whether the arrangement of the retaining magnetic field generator comprises at least one suitable saddle point. Moreover, such numerical simulations of the magnetic field generated by the retaining magnetic field generator can also be utilized to test whether a given arrangement of magnetic objects can be regarded as forming a magnetic field with a saddle point as defined above. However, instead of numerical simulations also experiments can be used that, for instance, measure a possible displacement of a magnetic object arranged in relation to other magnetic objects forming the retaining magnetic field generator to determine if the magnetic object is positioned at a saddle point formed by the retaining magnetic field generator.

In a preferred embodiment, the rotary bearing comprises a retaining surface in a spatial direction perpendicular to the predefined spatial plane such that a movement of the magnetic object in the perpendicular spatial direction is restricted. Thus, the retaining surface is arranged such that the magnetic object is retained substantially at the saddle point. Preferably, the retaining magnetic field generator is adapted to provide the retaining surface. This allows to provide the rotary bearing without additional components. However, the retaining surface can also be provided by additional elements that are arranged with respect to the retaining magnetic field generator such that the magnetic object is retained substantially at the saddle point. Since the saddle point provides a stable position in two dimensions but might not be able to stabilize the magnetic object in the third dimension, the retaining surface allows for a retaining of the magnetic object with respect to this third dimension at the saddle point. Preferably, the retaining surface is arranged and adapted such that the magnetic object touches the retaining surface as little as possible, more preferably at only one point. The term "one point" in this respect refers to a realistically reachable contact surface area between the retaining surface and the magnetic object. A realistically reachable contact surface area refers to an area smaller than 100 times a physically smallest possible contact area, preferably 10 times the physically smallest possible contact area, wherein the physically smallest possible contact area is determined by the physical laws acting on the contact surface area and depends on the materials used for the retaining surface and the magnetic object, the forces acting on the magnetic object in the direction of the retaining surface, and the geometry of the magnetic object.

The retaining surface comprises at least in a possible contact surface area preferably a compressive strength of more than 10 GPa. Moreover, it is preferred that the retaining surface comprises at least in a possible contact area a Young modulus of at least 10 GPa, more preferably at least 50 GPa. Materials comprising these characteristics are, for instance, glass, DLC, diamond, etc. Such materials can be provided at the retaining surface, for example, as layer over another material, at potential surface contact areas. However, the retaining surface can also be entirely made of one or more such materials.

In a preferred embodiment, the retaining magnetic field generator is adapted such that a normal force applied by the magnetic object on the retaining surface is lower than 1000 times, preferably 100 times, the gravitational force acting on the magnetic object, wherein the normal force is defined as a force acting along the perpendicular spatial direction on the surface. Generally, a normal force can occur at the contact point along the dimension that is not stable at the saddle point, i.e. the direction preferably perpendicular to the retaining surface. Decreasing the normal force allows to also decrease a friction between the magnetic object and the retaining surface, which leads to an increased Q-factor for the rotational motion of the rotating magnetic object. The Q-factor is a dimensionless parameter that describes how underdamped an oscillator or resonator is. It is defined as the ratio of the peak energy stored in the resonator in a cycle of oscillation to the energy lost per radian of the cycle, wherein the lower the Q-factor is the higher is the damping of the oscillation or rotation. Low normal forces, as preferred, can be achieved by arranging the magnetic field generator and the retaining surface accordingly, for instance, as described in the below embodiments. Moreover, simulations as already described for simulating a magnetic energy landscape of an arrangement of magnetic objects can also be used for simulating the normal forces acting on a retaining surface. Thus, if a suitable arrangement of magnetic objects providing a magnetic field with a saddle point is found, normal forces acting on retaining surfaces positioned for retaining the magnetic object can be simulated. Based on such simulations, parameters like a size of or a field strength provided by the retaining magnetic field generator can be optimized to optimize the normal force. Moreover, such simulations can also be used to test whether a given arrangement fulfills the requirement for the normal forces described above.

In a preferred embodiment, the retaining magnetic field generator comprises two retaining permanent magnetic objects arranged on opposite sides of the magnetic object such that the saddle point is provided between the retaining permanent magnetic objects. In a preferred embodiment the two retaining permanent magnetic objects each have a spheroidal, more preferably a spherical, shape. Providing the retaining permanent magnetic objects in form of permanent magnetic spheres allows for an easier production and arrangement of the retaining permanent magnetic objects. Alternatively, the retaining permanent magnetic objects can also have a cylindrical shape or the shape of a cuboid, wherein these shapes have the advantage that the retaining magnetic field generated by the two retaining permanent magnetic objects can be calibrated more accurately. In order to increase a stability of this arrangement, it is preferred that the retaining permanent magnetic objects are provided with a size greater than the rotating magnetic object, for instance, in case of a spherical shape, with a greater diameter than the rotating magnetic object. Preferably, the retaining permanent magnetic objects refer each to a neodymium magnet with a remanence larger than N52, i.e. larger than 1.42 T. In applications in environments with a temperature of more than 80°C it is preferred that the retaining permanent magnetic objects refer each to a neodymium magnet with H, SH, EH or AH characteristics according to the general neodymium magnet identification system. The H, SH, EH or AH type neodymium magnets are also advantageously employed for the retaining permanent magnetic objects in applications in which the magneto mechanical rotator is expected to be subjected to high magnetic fields, like present in magnetic resonance applications. Also CoSm (cobalt samarium) magnets can be employed as retaining permanent magnetic object.

In a preferred embodiment, the two retaining permanent magnetic objects are arranged such that each of the retaining permanent magnetic objects forms a retaining surface in the spatial direction perpendicular to the predefined spatial plane for restricting the movement of the magnetic object. Thus, the retaining permanent magnetic objects of the retaining magnetic field generator provide at the same time each a retaining surface, which allows for a much easier construction of the microdevice. It is preferred that the rotating magnetic object is arranged such that the perpendicular spatial direction refers to a rotation axis of the rotating magnetic object. However, in other embodiments the rotation axis of the rotating magnetic object can also refer to other directions, in particular, the rotation axis of the magnetic object can change with time. In a preferred embodiment, the two retaining permanent magnetic objects are arranged such that the magnetic moments of the two retaining permanent objects are arranged to stabilize a rotation of the magnetic object. In an embodiment, the retaining magnetic field generator can comprise one or more additional retaining permanent magnetic objects, in particular, additional retaining permanent magnetic objects smaller than the two retaining permanent magnetic objects, that allow to compensate small inaccuracies in the arrangement of the two retaining permanent magnetic objects and to determine an orientation of a rotation axis of the magnetic object.

Preferably, the retaining permanent magnetic objects and optionally the additional retaining magnetic objects are arranged such that the orientation of the rotation axis of the magnetic object changes less than a predetermined threshold. Preferably, the change of the orientation of the rotation axis is defined by a change, i.e. a movement, of a crossing point in space that is defined as one of the points at which the rotation axis crosses the surface of the rotating magnetic object, i.e. by a change in the position of the geographical pole of the magnetic object. Preferably, the retaining permanent magnetic objects and optionally additional retaining magnetic objects are arranged such that the geographical pole of the rotating magnetic object moves during a 10° rotation less than 2°, preferably less than 0.2°. Moreover, a stability of the rotation axis of the magnetic object can also be defined with respect to the contact point of the permanent magnetic object, i.e. with respect to the ideal point at which the permanent magnetic object contacts a retaining surface, and a contact area referring to the average realistic contact surface area between the rotating magnetic object and the retaining surface. With these two definitions the two retaining permanent magnetic objects and optionally the additional retaining permanent magnetic objects are arranged preferably such that during the rotation of the magnetic object the contact point moves less in space than ten times the contact surface area, preferably five times the contact surface area, more preferably less than the contact surface area. However, in other embodiments the two retaining permanent magnetic objects and optionally additional magnetic objects are arranged such that an abradant movement of the contact point is prevented, whereas a rolling movement is allowed.

Generally, a stabilization of the rotation of the magnetic object, in particular, with respect to a minimization of the movement of the contact point on the retaining surface allows to minimize frictional forces acting on the rotating magnetic object. The reduction of the frictional forces also minimizes a loss in the angular momentum and thus maximizes the Q-factor of the magneto mechanical rotator such that it can provide a more stable magnetic signal.

In this context, it is also preferred that the two permanent magnetic objects are arranged such that the orientations of the magnetic moments of the permanent magnetic objects are fixed relative to the casing over a time scale that is comparable with the time scale of the mechanical rotation of the magnetic object. Thus, the rotating magnetic object experiences a stable retaining magnetic field during the rotation of the magnetic object, wherein a general orientation of the magnetic field might change on longer time scales, for instance, to adapt to external magnetic influences like magnetic fields generated by a magnetic resonance device, etc.

In an embodiment, the retaining magnetic field generator additionally comprises two soft magnetic objects, wherein the two soft magnetic objects are arranged on the opposite sides of the magnetic object at which the retaining permanent magnetic objects are arranged, wherein the two retaining permanent magnetic objects are arranged further away from the magnetic object than the soft magnetic objects. It is further preferred that the retaining permanent magnetic objects and the soft magnetic objects are all arranged substantially along the rotation axis of the rotating magnetic object. Providing the additional soft magnetic objects allows for a complete rotational oscillation of the magnetic object. Preferably, the two soft magnetic objects are two soft magnetic discs, wherein the term "discs" can also refer to a cylindrical shape, and at least one of the flat surfaces provided by the soft magnetic discs is arranged as the retaining surface for the rotating magnetic object. Moreover, the two soft magnetic objects can comprise a concave shaped surface as retaining surface. In this embodiment, the soft magnetic objects can refer to a disc shape with at least one concave shaped surface and thus can be bowl shaped.

In an embodiment, the magneto mechanical rotator comprises an additional rotating magnetic object that is stabilized by the rotary bearing and is adapted to rotate relative to the rotary bearing. Preferably, the additional rotating magnetic object is similar to the magnetic object as already described above. Hence, it is preferred that the magneto mechanical rotator comprises two rotating magnetic objects that are stabilized by the rotary bearing and adapted to rotate relative to the rotary bearing. It is even more preferred that the rotation axis of the two rotating magnetic objects is the same. In particular, the rotation of the two rotating magnetic objects refers in this case to a rotational oscillation. Also in this embodiment, the rotary bearing can comprise a retaining magnetic field generator such that each of the magnetic objects can be regarded as being positioned substantially at a saddle point of the retaining magnetic field generator. In particular, if two rotating magnetic objects are present from the point of view of one of these rotating magnetic objects, the other rotating magnetic object can be regarded as part of the retaining magnetic field generator contributing to generating the saddle point on which the one rotating magnetic object is positioned. Due to the possible movements of the two rotating magnetic objects, the saddle point can be interpreted more dynamically. In particular, the saddle point in this case can be defined by the six degrees of translational freedom of the two rotating magnetic objects, wherein in this case the retaining magnetic field generator is adapted to provide a saddle point that restricts the translational freedom of the two magnetic objects in at least four of the degrees of freedom and wherein the retaining surface can be positioned in at least one of the directions of the still unrestricted degrees of freedom.

A medical microdevice comprising two rotating magnetic objects can in particular not only be utilized for localizing the medical microdevice but also as a sensor for sensing a physical parameter in the environment of the medical microdevice. In such an embodiment, the rotary bearing and the two rotating magnetic objects are arranged such that the environmental physical parameter is able to influence a magnetic interaction between the two rotating magnetic objects, for instance, by influencing a distance between the two rotating magnetic objects. The change in the magnetic influence of the two rotating magnetic objects on each other generally also influences the response magnetic field generated by the two rotating magnetic objects, for instance, by changing a frequency or by adding higher harmonics to the response magnetic field generated by the magneto mechanical rotator. These changes in the response magnetic field can then be measured, for instance, by the magnetic coils provided for localizing the medical microdevice, and can be analyzed to determine the change of the physical parameter in the environment of the medical microdevice. Moreover, baselines for physical parameters in the environment of the medical microsensor can also be measured, for instance, during a calibration measurement, and the physical parameter can then be determined based on this baseline measurement, for example, based on a comparison between a current measurement and the baseline. Thus, not only changes of a physical parameter, but also absolute values of the physical parameter can be determined based on the response magnetic field generated by the magneto mechanical rotator.

In a preferred embodiment, the rotary bearing for the two rotating magnetic objects is constructed as already described above. Preferably, the additional rotating magnetic object is arranged between the two retaining permanent magnetic objects such that a rotation axis of the two rotating magnetic objects is the same. Even more preferably, at the rotation axis on opposite sides of each of the magnetic objects soft magnetic discs are arranged, wherein the two retaining permanent magnetic objects are arranged further away from the magnetic objects along the rotation axis than the soft magnetic discs. The soft magnetic discs can refer to magnetic discs as already described above. It is preferred that the two retaining magnetic objects in this embodiment are provided smaller than the rotating magnetic objects, for instance, in case of spheres, with a smaller diameter than the diameter of the rotating magnetic objects. This has the advantage that the system becomes more sensitive to distance changes between the rotating magnetic objects and thus more sensitive for sensing a physical parameter change in the environment of the medical microdevice. Moreover, also a separation can be provided that is adapted to separate the two rotating magnetic objects such that they cannot directly contact each other. In an embodiment, the separation can be formed by one or both of the two soft magnetic disc described above. However, the separation can also be independent of the soft magnetic discs. For example, the separation can be provided by the casing or as part of the casing. Preferably, the separation is utilized as at least one retaining surface for one or both rotating magnetic objects.

In an embodiment, more than one retaining permanent magnetic object is arranged on each side of the magnetic object. The additional retaining permanent magnetic objects arranged on a side of the magnetic object comprise preferably the same shape, for instance, a spherical shape. However, in other embodiments the retaining permanent magnetic objects on one side of the magnetic object can also be different from each other, for instance, comprise different shapes. The retaining permanent magnetic objects on one of the sides of the magnetic object can be arranged along a common axis being preferably perpendicular to the main rotation axis of the rotating magnetic object. However, in other embodiments the retaining permanent magnetic objects can also comprise other arrangements with respect to each other. For stabilizing a desired arrangement of the more than one retaining permanent magnetic object, the permanent magnetic objects can be provided within a housing surrounding the arrangement of retaining permanent magnetic objects. However, the arrangement can also be stabilized using other elements than a housing, for instance, flexible or stiff connections between the retaining permanent magnetic objects on one side, etc. In a preferred embodiment, the more than one retaining permanent magnetic objects on a side of the magnetic object are arranged such that they are moveable with respect to each other. Preferably, the movement of the permanent magnetic objects allows the retaining permanent magnetic objects to rearrange the direction of the magnetic moments with respect to each other. It is thus preferred that the movement of the permanent magnetic objects with respect to each other refers to a free rotation of the retaining permanent magnetic objects, while the positions of the retaining permanent magnetic objects remain fixed with respect to each other. This arrangement leads to a pseudo-soft magnetic effect of the arrangement of the retaining permanent magnetic objects due to the ability of the retaining permanent magnetic objects to rearrange their magnetic moments in response to external magnetic influences similar to the rearrangement of magnetic areas in a soft magnetic material. Such an embodiment can be achieved, for instance, by providing the retaining permanent magnetic objects within a housing that constrains the movement of the retaining permanent magnetic objects with respect to each other to desired movements, for instance, to rotational movements. Allowing a movement of the retaining permanent magnetic objects with respect to each other, in particular, a rotation of the retaining permanent magnetic objects, allows the medical microdevice to adapt the magnetic field produced by the retaining permanent magnetic objects to external circumstances without destroying the device. For example, when subjected to a strong magnetic field like in a magnetic resonance environment the retaining permanent magnetic objects can adapt to this environment without damaging the medical microdevice, wherein if the external influence is removed, the retaining permanent magnetic objects can rearrange themselves again to form the retaining magnetic field. Preferably, a high viscous fluid is provided between the retaining permanent magnetic objects for dampening the movement of the retaining permanent magnetic objects. In particular, the highly viscous fluid can fill a housing in which the retaining permanent magnetic objects are arranged. Providing a highly viscous fluid between the retaining permanent magnetic objects on a side of the magnetic object for dampening the movement of the retaining permanent magnetic objects allows to stabilize the movement of the retaining permanent magnetic objects such that only external magnetic influences of a predetermined strength lead to a rearrangement of the retaining permanent magnetic objects, whereas small fluctuations in the magnetic environment of the medical microdevice will not have an influence on the retaining magnetic field generated by the retaining permanent magnetic objects. Highly viscous fluids generally comprise a viscosity of more than 8000 mPas. Preferably, the highly viscous fluid refers to a silicone oil, however also sugar solutions can be utilized. Generally, the highly viscous fluid can be selected depending on the intended application of the magneto mechanical rotator. For example, if the magneto mechanical rotator shall be used also in an environment of a magnetic resonance imaging system comprising a typical magnetic flux density of 1 T, providing a fluid with a viscosity between 10 kPas and 1000 kPas allows the retaining permanent magnetic objects to orient themselves in the external magnetic field without taking damage.

In an embodiment, the retaining magnetic field generator comprises a retaining permanent magnetic object and a retaining soft magnetic object. Preferably, the retaining permanent magnetic object and the retaining soft magnetic object are arranged on opposite sides of the rotating magnetic object. Further, it is preferred that the retaining permanent magnetic object has a spheroidal shape, whereas the soft magnetic object has a disc shape, wherein also in this case a disc shape can encompass a cylindrical shape or a bowl shape. Preferably, one of the planar surfaces provided by the disc shape of the retaining soft magnetic object forms the retaining surface for retaining the rotating magnetic object. Also this arrangement allows for a good stabilization of the rotation axis of the rotating permanent magnetic object and is, furthermore, easy to construct.

In an embodiment, the retaining magnetic field generator comprises two soft magnetic objects, preferably, two soft magnetic discs, arranged on opposite sides of the magnetic object such that the saddle point is provided between the soft magnetic objects. The two soft magnetic objects can have any shape, for instance, can be formed by a soft magnetic sheet. However, preferably the two soft magnetic objects refer to two soft magnetic discs. Also in this embodiment, the term "disc" can encompass cylindrical shapes and bowl shapes. Preferably, the soft magnetic discs are arranged such that the flat surfaces provided by the soft magnetic discs are perpendicular to a rotation axis of the rotating magnetic object such that the soft magnetic discs are arranged at the rotation axis of the rotating magnetic object. In a preferred embodiment, at least one of the planar surfaces provided by the two soft magnetic discs forms the retaining surface. However, the two soft magnetic discs can also comprise a concave surface area, wherein this area can provide the retaining surface. For example, the soft magnetic discs can comprise a bowl like shape or a concave indention in one of the planar surfaces. Preferably, the soft magnetic discs can be formed from or can comprise any soft magnetic material. Preferably, the soft magnetic discs comprise any one of electric steel, nickel based materials, and amorphous or nano-crystalline materials.

In a preferred embodiment, the rotary bearing further comprises a supplemental magnetic ring arranged around the magnetic object and in between the two soft magnetic discs, wherein the supplemental magnetic ring is arranged in a plane parallel to the plane provided by the planar surfaces of the soft magnetic discs. In this embodiment, the term "arranged in a plane" used with respect to the supplemental magnetic ring defines that at least three points of the magnetic ring have to lie within the respective plane. In particular, the supplemental magnetic ring comprises soft magnetic material and is arranged in the middle between the two soft magnetic discs. Thus, the ring is arranged preferably around an equatorial region of the rotating magnetic object. The supplemental magnetic ring allows for a further stabilization of the rotation axis of the rotating magnetic object when the rotating magnetic object is subjected to external fluctuating magnetic fields.

In a preferred embodiment, the rotary bearing comprises at least one additional soft magnetic sheet on each side of the magnetic object at which a retaining magnetic disc is arranged, wherein the planar surfaces of the additional magnetic sheets are arranged parallel to the planar surfaces of the retaining magnetic discs. The additional magnetic sheets can have any shape, for instance, a circular or quadratic shape. Preferably, the magnetic sheets comprise a thickness of less than 1/10 of a lateral dimension of the sheet. The lateral dimensions of the sheets are preferably less than 40 %, more preferably less than 20 %, of the diameter of the magnetic object. Preferably, the additional magnetic sheets have the form of additional magnetic discs arranged parallel to the planar surfaces of the retaining magnetic discs. Thus, preferably at least two soft magnetic discs are arranged on opposite sides of the magnetic object. The additional soft magnetic discs have preferably a smaller diameter than the retaining magnetic discs. Generally, the one or more magnetic discs arranged on opposite sides of the magnetic object are provided with a diameter and a distance to each other such that a retaining magnetic field with the desired saddle point is provided and such that a desired stabilization of the rotation axis of the rotating magnetic object is achieved.

Also for the above described embodiments comprising a retaining permanent magnetic object and a retaining soft magnetic disc, or two retaining soft magnetic discs, an additional rotating magnetic object can be provided in between as already described above. Also in these embodiments, the additional magnetic object, i.e. the provided two rotating magnetic objects, allow the utilization of the medical microdevice as sensor for sensing a physical parameter in the environment of the medical microdevice, for instance, by changing a distance between the two rotating magnetic objects based on the change of a physical parameter in the environment. Alternatively, each of the two rotating magnetic objects can be provided with its own rotary bearing, for example, with its own retaining soft magnetic discs.

In a preferred embodiment, the medical microdevice comprises two rotating magnetic objects each comprising its own rotary bearing, wherein the rotary bearings associated with each of the two rotating magnetic objects are similar to each other. Thus, it is preferred that both rotary bearings comprise, for instance, two permanent magnetic spheres as retaining magnetic field generator, or two retaining soft magnetic discs as retaining magnetic field generator. This embodiment can be regarded as referring to a medical microdevice comprising two magneto mechanical rotators in the casing. Preferably, each of these magneto mechanical rotators is provided in its own sub-casing such that they cannot directly physically contact each other. Moreover, providing a sub-casing for each magneto mechanical rotator has the advantage that the influence of a physical parameter in the environment of the medical microdevice can be provided to at least one of the sub-casings, for instance, by changing a position of one of the sub-casings relative to the other one of the sub-casings. In a particularly preferred embodiment, one of the sub-casings can be connected to a kind of bellows or elastic foil that allows the change of a distance between the sub-casings based on a pressure change in the environment of the medical microdevice. However, in other embodiments the sub-casings can be omitted and the two magneto mechanical rotators can be otherwise at least partially connected to the casing of the medical microdevice, for instance, by utilizing the rotary bearings to position and connect the magneto mechanical rotators within the casing.

In an embodiment, the rotary bearing comprises a flat surface and a magnetic foil arranged underneath and parallel to the flat surface such that a magnetization direction of the magnetic foil is perpendicular to a rotation axis of the magnetic object. Also in this embodiment, a position and rotation axis of the rotating magnetic object can be stabilized by the magnetic field provided by the magnetic foil arranged underneath the surface.

In an embodiment, the rotary bearing comprises a lubricant fluid adapted to reduce friction between surfaces of the rotary bearing and the magnetic object. Providing a lubricant fluid for the reduction of friction between the surfaces of the rotary bearing and the magnetic object, in particular, between the surfaces at contact areas between the rotary bearing and the magnetic object, allows for a longer and more stable rotation of the rotating magnetic object once it has been excited by the excitation field such that the magneto mechanical rotator provides an improved magnetic signal that can be measured for the localization of the medical microdevice. Generally, the reduction of the friction between the surfaces of the rotary bearing and the magnetic object relates to a reduction with respect to a rotary bearing that does not comprise a lubricant fluid. Preferably, the lubricant fluid is a ferrofluid. Utilizing a ferrofluid has the advantage that the magnetic object will be suspended by the ferrofluid and thus not be in direct contact with the retaining surface leading to a further decrease of the friction experienced by the magnetic object. However, also other lubricant fluids can be utilized. For example, water, ether or alcohol can be used as lubricant fluid. However, in this case the corrosive characteristics of these fluids are preferably considered, for instance, by providing surfaces in contact with these fluids with an anticorrosive layer. Alternatively, the lubricant fluid can also refer to a fluid comprising mainly short alkanes, like pentane, that are not corrosive with respect to the magnetic object or the retaining surface.

In an embodiment, the medical microdevice further comprises a signal modulator, wherein the signal modulator is adapted to modulate a response magnetic field generated by the magneto mechanical rotator when excited, wherein the modulated magnetic field allows for a localization of the device. Generally, the signal modulator can refer to any structure, in particular, to any hard or soft magnetic structure that has a predetermined influence on the response magnetic field that is generated by the magneto mechanical rotator after the exciting phase, i.e. during the free rotation of the magneto mechanical rotator. The signal modulator is adapted such that its influence on the response magnetic field refers to a periodic influence and thus to a modulation of the response magnetic field. Modulating the response magnetic field provided by the magneto mechanical rotator has the advantage that without increasing the rotational speed of the magneto mechanical rotator a magnetic signal with a higher frequency can be provided which allows for a more accurate and easier localization of the device. Moreover, the modulation of the response magnetic field allows the usage of a plurality of medical microdevices that are excited by the same excitation field and thus would substantially provide the same response magnetic field. By applying a modulation that differs for each medical microdevice, the different response magnetic fields provided by the different medical microdevices can be distinguished and localized individually. Preferably, the signal modulator is provided in embodiments in which the rotating magnetic object of the magneto mechanical rotator transduces the excitation field into a complete rotational motion, i.e. a repeated rotation of the magnetic object around 360° in the same direction. For example, it is advantageous to provide a signal modulator to the embodiments described above comprising one rotating magnetic object and a retaining magnetic field generator that allows for such a complete rotation, for instance, a retaining magnetic field generator comprising one or more soft magnetic discs. If the magneto mechanical rotator is only applied for localization purposes, it is preferred that the signal modulator refers to a soft magnetic band arranged at a fixed position such that a modulation of the response magnetic field of the rotating magnetic object is provided. Alternatively, a permanent magnet can also be arranged at a fixed position such that a modulation of the response magnetic field of the rotating magnetic object is provided. Depending on the position and arrangement of the soft magnetic band or the permanent magnetic object, the modulation characteristics can be adjusted individually for each magneto mechanical rotator such that different magneto mechanical rotators can be differentiated by their differently modulated signal.

Preferably, the signal modulator comprises at least a part with a soft magnetic material. Providing the signal modulator at least partially with a soft magnetic material that allows for a magnetization and demagnetization caused by the response magnetic field generated by the magneto mechanical rotator allows for a very effective modulation of the generated response magnetic field.

In an embodiment, the signal modulator is adapted such that changes of a physical parameter in the environment of the medical microdevice introduce changes of the modulation of the response magnetic field that allow a determination of the changes of the physical parameter from a measurement of the modulated magnetic field. Thus, in this embodiment the medical microdevice can be utilized as a sensor for sensing changes of a physical parameter in its environment. The physical parameters can refer to any physical parameters in the environment, for instance, temperature, pressure, humidity, etc. For example, a part of the signal modulator can comprise a polymer that changes its characteristic, for instance, its extent, based on a presence of water in an environment. Such a change allows for a humidity determination in the environment of the medical microdevice based on the changed modulation of the magnetic signal. Moreover, the physical parameter can also refer to a chemical parameter. For example, the signal modulator can be adapted to change a characteristic based on the presence or concentration of a specific substance in the environment of the medical microdevice, wherein the changing characteristic leads to a change in the modulation of the response magnetic field.

In this embodiment, the casing can be specifically adapted to allow for a transfer of the changes of the physical parameter in the environment of the medical microdevice to the signal modulator. For example, the casing can be provided with a structure, for instance, an elastic membrane, that changes a characteristic due to changes of a physical parameter, like temperature or pressure, in the environment of the medical microdevice. The signal modulator might then react to the changes of this structure of the casing, for instance, the signal modulator can be connected to an elastic membrane such that a distance to the magneto mechanical rotator changes if a pressure in the environment of the medical microdevice changes. However, in other embodiments the signal modulator can directly react to the changes of the environment, for instance, by changing one or more of its characteristics, for example, due to a temperature change in the environment of the signal modulator.

In an embodiment, the signal modulator is adapted such that changes of the physical parameter lead to changes of the internal structure of the signal modulator such that the changes of the internal structure of the signal modulator introduce changes of the modulation of the response magnetic field. For example, in a preferred embodiment the physical parameter refers to a temperature in the environment of the medical microdevice and the signal modulator is adapted such that the internal structure change of the signal modulator refers to a change of a magnetic saturation of the signal modulator in dependence on the temperature. The signal modulator in this embodiment can comprise at least a soft magnetic part, wherein the soft magnetic part changes a magnetic saturation based on the physical parameter. If temperature changes lead to a change in a magnetic saturation of the signal modulator, the influence of the signal modulator on the generated response magnetic field also changes in dependence on the temperature. Thus, the modulated signal allows to deduce the change of the temperature experienced by the medical microdevice, for instance, by recognizing such a change in the modulation of the generated magnetic signal or by utilizing a comparison between a current modulation of the generated response magnetic field and a modulation of the generated response magnetic field measured during a calibration measurement at a predetermined temperature. Advantageously, the signal modulator comprises at least a part comprising a soft magnetic material with a Curie temperature lying within a predetermined temperature range including an expected measurement temperature range. Thus, the soft magnetic material provided in the signal modulator can be chosen based on the temperature range that is expected in the environment in which the medical microdevice shall be applied. Preferably, if the medical microdevice shall measure a temperature inside a human being, the soft magnetic material can be chosen such that its Curie temperature lies within a temperature range between 36°C and 41°C plus a respective tolerance of preferably 20°C, more preferably 50°C, for instance, within a temperature range between 16°C and 61°C. It has been shown that such tolerances do not have an influence on the quality of the temperature measurement. Accordingly, for the desired application of the medical microdevice different versions of the medical microdevice can be provided.

In a preferred embodiment, the physical parameter refers to a temperature in the environment of the medical microdevice and the signal modulator is adapted such that the internal structure change of the signal modulator refers to a change of a magnetic remanence of the signal modulator in dependence on the temperature. Also in this case, the influence of the signal modulator depends on the temperature. Based on the intended application, materials with different functional relationships between the remanence and the temperature can be employed.

In an embodiment, the signal modulator comprises a mechanical resonator, wherein the mechanical resonator can be excited by the response magnetic field generated by the rotational movement of the magnetic object, wherein the mechanical resonator is adapted such that changes of the physical parameter lead to a change of the resonance frequency of the mechanical resonator such that the excitation of the mechanical resonator by the generated response magnetic field introduces changes of the modulation of the response magnetic field in dependency of the physical parameter. Thus, the mechanical resonator is adapted such that the response magnetic field generated by the rotational movement of the magnetic object generally allows for an excitation of the mechanical resonator. Such an excitation of the mechanical resonator thus leads to the modulation of the generated response magnetic field due to the superposition of the generated response magnetic field and the magnetic field generated by the mechanical resonator when excited. The mechanical resonator is then adapted such that the change of the physical parameter in the environment of the medical microdevice leads to a change of the resonance frequency of the mechanical resonator. The resonance frequency can change such that the mechanical resonator becomes more susceptible to the generated response magnetic field, for instance, if the generated response magnetic field before the change of the physical parameter was not able to substantially excite the mechanical resonator. However, the resonance frequency can also change such that the mechanical resonator can be excited by the generated response magnetic field, or such that after the change of the physical parameter the mechanical resonator becomes less susceptible to the generated response magnetic field, i.e. less excitable. It is therefore preferred that the mechanical resonator is adapted such that the resonance frequency of the mechanical resonator corresponds to the frequency of the response magnetic field generated by the rotating magnetic object when the physical parameter lies in an expected range of the physical parameter for the specific application of the medical microdevice.

In a preferred embodiment, the mechanical resonator comprises a) a magnetic component that is adapted to change an internal structure in dependency of an influence of the magnetic object and b) a bellows that is adapted to provide a restoring force against the change of the internal structure. Generally, the bellows refers to a flexible structure that can react to forces by changing one of its characteristics, for instance, by bending, by shortening or by expanding, wherein the changes of the characteristics of the bellows then lead to a restoring force acting against these changes of the characteristics of the bellows. Since changes in the characteristics of the bellows refer also to changes in the internal structure of the mechanical resonator, the bellows provides a restoring force against such changes of the internal structure. The interaction between the external forces, in particular, the magnetic forces provided by the generated response magnetic field acting on the magnetic component of the mechanical resonator and the restoring forces provided by the bellows introduce an oscillation of the mechanical resonator around a state of equilibrium and thus lead to an excitation of the mechanical resonator. The resonance frequency of the mechanical resonator can then be changed, for instance, by introducing a change in the magnetic component or by introducing a change in the restoring force provided by the bellows. For example, the external physical parameter can have an influence on the magnetization of the magnetic component leading to differences in the change of the internal structure in dependency of the influence of the magnetic object on the magnetic component. In particular, a change in the magnetization leads to a change in the resonance frequency of the mechanical resonator. In another example, the change of the characteristics of the bellows caused by the change of the internal structure can depend on the physical parameter such that also the restoring force provided by the bellows against the change of the internal structure changes, which also can lead to the change of the resonance frequency.

In a preferred embodiment, the magnetic component comprises a soft magnetic element and a flux concentrator arranged with a predetermined offset and at a distance to each other such that the resonance frequency of the mechanical resonator depends on the distance between the soft magnetic element and the flux concentrator, wherein the bellows is adapted such that based on a change of the physical parameter the distance between the soft magnetic element and the flux concentrator is changed, wherein the flux concentrator refers to a rigid structure of a soft magnetic material arranged such that it amplifies the response magnetic field and the field gradient provided by the magnetic object at the location of the signal modulator. For example, the bellows can be provided such that a change in a pressure compresses or expands the bellows and changes the equilibrium distance between the soft magnetic component and the flux concentrator. This change in the equilibrium distance then leads to a different oscillation, i.e. an oscillation around a different equilibrium point, which at the same time leads to a different resonance frequency. The offset of the soft magnetic component and the flux concentrator refers to a distance between the surfaces with which the flux concentrator and the soft magnetic component face each other in a direction perpendicular to the distance between the soft magnetic component and the flux concentrator. Preferably, the offset with which the flux concentrator is arranged relative to the soft magnetic component refers to a diameter of the surface with which the flux concentrator faces the soft magnetic component. For example, the offset can be 50 $\mu$m if the diameter of the surface with which the flux concentrator faces the soft magnetic component also comprises 50 $\mu$m. The offset allows for transferring changes of the magnetic flux, in this case, changes in a magnetic environment, more effectively into an oscillation of the mechanical resonator.

In an embodiment, the magnetic component comprises at least two magnetic elements of soft magnetic material in form of tuning forks arranged such that prongs of the tuning forks face each other and such that the resonance frequency of the mechanical resonator depends on the distance between the prongs of the tuning forks, wherein the bellows is adapted such that based on a change of the physical parameter the distance between the two magnetic tuning forks is changed. The form of a tuning fork refers to a form comprising two or more prongs and a component that connects the two or more prongs. Thus, the reference to the magnetic elements as tuning forks refers to their geometric form not to their function. The functional principles of this embodiment of the magnetic resonator are similar to the functional principles explained above with respect to the magnetic component comprising a soft magnetic component and a flux concentrator. In particular, one of the two magnetic parts in form of a tuning fork can also be regarded as a specific flux concentrator and the other as a specific soft magnetic component. Preferably, the at least two tuning forks are arranged such that the prongs are offset to each other by a predetermined distance with respect to a direction perpendicular to the prongs. Preferably, also in this embodiment the offset refers to a diameter of the prongs.

In an alternative embodiment, friction bearings could be used for measuring the previously indicated parameters. In this embodiment, exemplary three magnetic objects could be used. Preferably, the magnetic objects are placed next to each other and in some embodiments, they can be separated by glass plates. Preferably, the central magnetic object is smaller than the other magnetic objects. In an exemplary non-limiting embodiment, the magnetic object at the center (inner magnetic object) could have a diameter of 0.5 mm and the other magnetic objects (outer magnetic objects) could have a diameter of 0.63 mm. The diameter of the inner objects could vary from 0.3 mm to 1 mm and the diameter of the outer objects could vary from 0.4 mm to 1.5 mm. In operation, the oscillations can be started in repeated pulse trains with high signal amplitude.

In an embodiment, the signal modulator is adapted such that changes of the physical parameter lead to a change of a distance between at least some part of the signal modulator and the magnetic object. For example, a signal modulator can be in contact with or can comprise a part of the casing that compresses or expands when subjected to more or less pressure in the environment of the medical microdevice. Moreover, also a temperature in the environment of the medical microdevice can influence, for instance, a length of an assembly attaching the signal modulator to the casing or to some other structure within the casing such that the distance between the signal modulator and the magnetic object changes. In a preferred embodiment, the signal modulator comprises a soft magnetic foil, wherein a change of the distance leads to a change of the magnetization of the soft magnetic foil. In particular, the soft magnetic foil is arranged such that the planar surface of the foil faces the rotating magnetic object. Preferably, the soft magnetic foil refers to an asymmetric soft magnetic foil, i.e. to a magnetic foil with a higher extent in one direction than in a perpendicular direction, for instance, to a soft magnetic foil with a rectangular shape or an elliptic shape. Preferably, the soft magnetic foil comprises a high aspect ratio, wherein high in this context is regarded as an aspect ratio above 1:5, preferably 1:7, and more preferably 1: 10. In a preferred embodiment, the demagnetization factor of the soft magnetic foil is smaller than 0.5, preferably smaller than 0.1. Such a demagnetization factor can, for instance, be reached with a high aspect ratio. However, such a small demagnetization factor can also be reached through other measures like the selection of the soft magnetic material, or other characteristics of the form of the soft magnetic foil. In another embodiment, the signal modulator can also comprise a shape of a cylindrical bar, wherein in this case a rounded side of the soft magnetic cylindrical bar faces the rotating magnetic object. Also in this case, a demagnetization factor of the cylindrical bar below 0.5, preferably below 0.1, is advantageous. In this case, such a small demagnetization factor can be reached, for instance, by selecting the cylindrical bar such that the length of the bar is longer than the diameter of the bar.

In an embodiment, the signal modulator further comprises a flux concentrator referring to a rigid structure of a soft magnetic material arranged such that it amplifies the response magnetic field and the response magnetic field gradient provided by the magnetic object at the location of the signal modulator.

In a further aspect of the invention a usage of the medical microdevice for localizing an object to which the medical microdevice is attached and/or for sensing a physical parameter in an environment of an object to which the medical microdevice is attached is presented. Preferably, the physical parameter refers to a temperature or pressure and the object refers to a device for being used in a human or animal body, in particular, an implantable device. In a preferred embodiment, the object refers to a guidewire or catheter. In another preferred embodiment, the medical microdevice is adapted to sense a blood pressure and is provided as part of a cardiac valve. Moreover, the medical microdevice can also be adapted to be or be part of a pulmonary artery pressure sensor.

In another preferred embodiment, the medical microdevice is adapted to sense as physical parameter an ionizing radiation and to be implantable as radiation measurement device into a human being or animal. A radiation sensitivity of the medical microdevice can be provided, for instance, by providing the medical microdevice with a casing comprising a material that is polymerized by the respective radiation and thus leads to a volume change of the casing. The magneto mechanical rotator of the medical microdevice can then be adapted, for instance, by using a signal modulator like described above, to modulate the signal of the magneto mechanical rotator based on the volume change of the casing.

In a further aspect of the invention a reading system for wirelessly reading out the medical microdevice as described above is presented, wherein the reading system comprises a) a field generator for generating a magnetic or electromagnetic excitation field for inducing a mechanical rotation of a magnetic object of a magneto mechanical rotator of the medical microdevice, wherein the rotation of the magnetic object generates a periodically changing response magnetic field, b) a transducer for sensing and transducing the generated response magnetic field into electrical response signals, and c) a processor for processing the electrical response signals. Generally, the field generator can refer to a field generator that can generate a periodically changing magnetic or electromagnetic field of a predetermined frequency. The transducer can refer to one or more magnetic coils that allow to transduce a magnetic field into an electric current that can be regarded as the electrical response signal. Preferably, the processor is adapted to determine a location and/or a physical parameter and/or a change of a physical parameter in an environment of the medical microdevice based on the electrical response signals. In a preferred embodiment, the field generator comprises at least one air-core coil that is adapted to generate an excitation field between 2 kHz and 200 kHz, wherein the transducer is preferably adapted to sense and transduce a magnetic signal of up to more than twice the frequency of the excitation field. Preferably, the transducer comprises at least one, preferably more than three, air-core coils made from copper or aluminum.

In a further aspect of the invention a method for localizing a medical microdevice as described above and/or for determining a physical parameter in the environment of a medical microdevice as described above is presented, wherein the method comprises a) generating a magnetic or electromagnetic excitation field for inducing a mechanical rotation of a magnetic object of a magneto mechanical rotator of the medical microdevice, wherein the rotation of the magnetic object generates a periodically changing response magnetic field, b) sensing and transducing the generated response magnetic field into electrical response signals, and c) processing the electrical response signals to determine a location and/or a physical parameter and/or a change of a physical parameter in an environment of the medical microdevice based on the electrical response signals.

In another aspect of the invention a computer program is presented, wherein the computer program comprises program code for causing a reading system as defined above to carry out the steps of the localization method and/or the measurement method as defined above, respectively, when the computer program is run on a computer controlling the reading system.

It shall be understood that the medical microdevice as described above, the reading system as described above, the method as described above and the computer program as described above have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

Fig. 1 shows schematically and exemplarily an embodiment of a medical microdevice comprising a magneto mechanical rotator,

Fig. 2 shows schematically and exemplarily a model for calculating lubrication in the medical microdevice,

Fig. 3 to Fig. 15 show schematically and exemplarily different advantageous embodiments of the medical microdevice, and

Fig. 16 shows schematically and exemplarily a flowchart of a method for measuring a magnetic signal of the medical microdevice.

DETAILED DESCRIPTION OF EMBODIMENTS

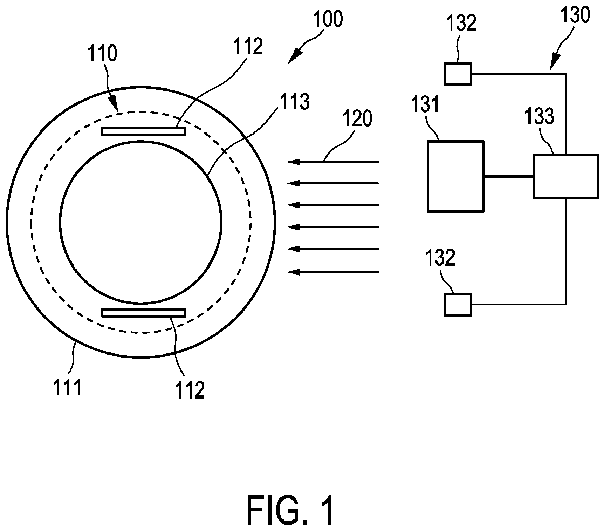

Fig.1 shows schematically and exemplarily an embodiment of a medical microdevice comprising a magneto mechanical rotator. The medical microdevice 100 comprises a casing 111 and within the casing the magneto mechanical rotator 110. The magneto mechanical rotator 110 is arranged within the casing 111, for instance, by providing the magneto mechanical rotator within an individual encasing structure that can also be a structural part of the casing 111 as indicated by the dashed line in Fig. 1. However, the magneto mechanical rotator 110 can also be arranged in the casing 111 by providing an attachment structure that allows to attach and stabilize the rotary bearing 112 in a stable position within the casing 111. The attachment structure can comprise an adhesive for gluing the rotary bearing 112 and/or other components of the magneto mechanical rotator 110 to the casing. Alternatively, the casing can comprise attachment structures that function as clamps for clamping the rotary bearing 112 and/or other components into place. The magneto mechanical rotator 110 comprises a permanent magnetic object 113 and the rotary bearing 112 that is adapted to stabilize the rotational motion of the magnetic object 113. In the embodiment shown in Fig. 1, the permanent magnetic object 113 provides a permanent magnetic moment indicated in Fig. 1 and in the following by the arrow within the magnetic object 113. Moreover, in this embodiment the magnetic object 113 is depicted as a sphere. However, also other shapes of the magnetic object 113 can be utilized advantageously. For example, prolate or oblate spheroids can also be employed. It is particularly preferred that the magnetic object 113 is adapted such that it provides the maximal moment of inertia around its rotation axis. This can be achieved, for instance, by choosing a corresponding shape or by adapting a mass distribution within the magnetic object accordingly.

The rotary bearing 112 in this embodiment comprises two magnetic discs that are arranged on opposite sides of the magnetic object 113 such that the magnetic moment of the magnetic object 113 can arrange itself in particular parallel to the surface that is provided by the magnetic discs. This kind of rotary bearing 112 is only one possibility for stabilizing the magnetic object 113 during its rotational motion while at the same time allowing for a rotational motion relative to the rotary bearing 112.

Generally, it is preferred that the rotary bearing 112 comprises a retaining magnetic field generator which in the example shown in Fig. 1 is formed by the two soft magnetic discs arranged around the magnetic object 113. The retaining magnetic field generator is adapted to generate a retaining magnetic field that provides a saddle point for the degrees of motional freedom of the magnetic object 113. Such a saddle point is defined such that it impedes the translational movement of the magnetic object 113 in two dimensions away from the saddle point when the magnetic object 113 is placed substantially at the saddle point. In particular, a magnetic restoring force is provided by the retaining magnetic field generator, in this example, the two soft magnetic discs, when the magnetic object 130 moves away from the saddle point in one of these dimensions, i.e. in a spatial plane pre-defined with respect to the rotary bearing 112. Thus, the magnetic field providing the saddle point is chosen such that it stabilizes the position of the magnetic object 113 in at least two dimensions. However, in the third dimension and thus in the third degree of translational freedom of the magnetic object 113 the magnetic field providing the saddle point might not provide a stabilizing, i.e. magnetic restoring force.

Thus, it is preferred for embodiments comprising a retaining magnetic field generator providing a magnetic field, as described above, that further a retaining surface is provided that retains the movement of the magnetic object 113 according to the third degree of translational freedom of the magnetic object 113, i.e. in a spatial direction perpendicular to the predefined spatial plane and thus perpendicular to the directions in which the movement of the magnetic object 113 is impeded. Generally, it is preferred that the retaining magnetic field generator, as shown in the exemplary embodiment of Fig. 1, also provides the retaining surface which can be in direct contact with the magnetic object 113.

The rotary bearing 112 shown in Fig. 1 is, as already discussed above, realized by two soft magnetic discs. Due to shape anisotropy, the soft magnetic discs prefer to be magnetized in the disc plane. This allows for a good alignment of the magnetic object 113 with the disc surfaces. In particular, if the saddle point is provided in the middle of the device, the magnetic alignment is as shown in Fig. 1. But, for this embodiment the true stable position is slightly off-center and the orientation not perfectly parallel nor perpendicular to the soft magnetic discs. However, if an external magnetic field 120 is provided, for instance, an external excitation field that is used for exciting, i.e. rotating, the magnetic object 113, the center position and alignment are stabilized. Once the magnetic object 113 rotates sufficiently fast, the center position becomes the stable position.

Generally, also for all following described embodiments of the rotary bearing comprising a retaining magnetic field generator, the retaining magnetic field and the saddle point can be calibrated as follows. For example, if the retaining magnetic field generator is realized in form of soft or permanent magnetic objects, the soft or permanent magnetic objects can be provided together with the magnetic object in a casing, for example, a tube, in accordance with the desired design. To manipulate a magnetization of one of the objects, for example, a laser or a heat generator, can then be utilized to adapt a distance and magnetic field characteristic of the magnetic system formed by the three or more magnetic objects. Alternatively, the soft or permanent magnetic objects of the arrangement can be mechanically manipulated, for instance, sanded in order to manipulate the magnetization and general magnetic field provided by the magnetic object. After having reached a desired configuration, the magnetic objects can be fixed in this configuration, for instance, by gluing them to the casing, and the casing can be closed after optionally having been filled with a lubrication fluid. Alternatively, a generally suitable configuration can be provided with one or more additional magnets outside the casing, for instance, glued or otherwise attached to the casing from the outside. Such additional magnets outside the casing allow for a correction and/or calibration of the magnetic field generated by the magnetic objects within the casing based on the location and orientation of the additional magnets outside the casing. Since this possibility can be used for calibrating the medical microdevice after having already provided a generally functional magneto mechanical rotator, it is much easier to perform.

Generally, for the calibration process a parameter is selected that can be measured and that should be optimized by the calibration process. Typically, the parameter refers to an oscillation frequency, if the magnetic object performs an oscillatory rotation, and/or to a quality factor of the rotation or oscillation. Based on the measurements of these parameters the above described methods can be used to manipulate the configuration of all magnetic components such that a desired parameter or parameter range is reached. The such found configuration can then be fixed, for instance, by gluing the components to the casing. The exact configuration can then be used for producing similar magneto mechanical rotators. Moreover, numerical simulations of the configurations of the magnetic components can be utilized for supporting and accelerating the calibration process.

Fig. 1 shows further a reading system 130 for wirelessly reading out the medical microdevice 100. The reading system 130 comprises at least a field generator 131 for generating the magnetic or electromagnetic excitation field 120 that can be used to excite the magneto mechanical rotator 110. Further, the reading system 130 comprises one or more transducers 132 that can measure the generated response magnetic field of the magneto mechanical rotator and transduce the generated response magnetic field into an electrical response signal. In some embodiments, the field generator 131 can also be utilized as transducer in addition or alternatively to the transducers 132 shown in Fig. 1. The transducers 132 can be realized as coils transducing the response magnetic field generated by the medical microdevice 100 into an electrical response signal. The electrical response signal can then be provided to a processor 133 that is adapted to process the electrical response signals, for instance, for localizing the medical microdevice or for determining a physical parameter measured by the medical microdevice in its environment. In particular, the processor 133 can be adapted to compare the response signal of different transducers and based on this comparison use a triangulation algorithm to determine the location of the medical microdevice. Moreover, the processor 133 can be adapted to analyze a frequency spectrum of the response signal and to compare the frequency spectrum to a frequency spectrum that is stored already, for instance, from a previous time span or from a calibration measurement. Based on this comparison the processor 133 can then determine a physical parameter or a change in a physical parameter in the environment of the medical microdevice. However, also other methods can be utilized for localizing the medical microdevice and/or for determining a physical parameter based on the response signals.

It is generally preferred for all embodiments of the medical microdevice that the minimal system Q-factor for the rotating magnetic object 113 is higher than 100. Since for embodiments provided with a retaining magnetic field generator, like the embodiment shown in Fig. 1, the position of the magnetic object 113 is mainly stabilized by magnetic forces, the only source of friction that might impact the system Q-factor is the contact between the retaining surface and the magnetic object 113. Thus, by optimizing this contact, for instance, by minimizing forces acting on this contact point and/or the friction at this contact point, the system Q-factor can be advantageously improved further.

Some general considerations for decreasing the friction at the contact point between the magnetic object and the retaining surface will be discussed in the following. Generally, the friction depends on a contact area between the magnetic object and the retaining surface. Thus, it is preferred that the contact area is kept near the physically smallest possible contact area. A physically smallest possible contact area for a magnetic object realized as a sphere can be determined, for instance, according to the following derivation. A normal force with respect to the contact surface resulting from magnetic forces can be determined by $F_m = \frac{4}{3}r^3\pi\rho g n$, wherein $r$ is the radius of the magnetic object being in this case a sphere, $\rho$ is the density of the magnetic object, $g$ is the gravitational acceleration, and $n$ is the ratio between the normal force of the magnetic object and the weight of the magnetic object. The normal force resulting from the counter pressure in the material is given by $F_p = PA = PR^2\pi$, wherein $P$ refers to the material hardness, $A$ is the physically smallest possible contact area, and $R$ is the physically smallest possible contact radius for a spherical magnetic object. Since both forces will equal each other, for the physically smallest possible contact radius it can be determined that $R = \sqrt{\frac{4}{3P}r^3\pi\rho g n}$. For example, for a contact surface comprising diamond-like carbon (DLC) with $P = 100$ GPa, a magnetic object comprising a density of $\rho = 7500$ kg/m$^3$, and a radius of $r = 250$ $\mu$m, the physical physically smallest possible contact radius refers to $R = 39$ nm. For a contact surface comprising steel or glass with approximately $P = 10$ GPa, the physically smallest possible contact radius refers to $R = 124$ nm. A realistically possible and suitable contact area refers to about 100 times, preferably 10 times, the physically smallest possible contact area. The torque acting on the rotating magnetic object caused by the friction, if a constant pressure is assumed on the contact surface, can be determined by $T = \int_0^R (2P\mu\pi x^2)\,dx = \frac{2}{3}P\mu\pi R^3$, wherein $\mu$ is the friction coefficient. Moreover, $T$ is also given as $T = I \cdot \omega$, wherein $I$ refers to the moment of inertia and $\omega$ to the angular velocity of the magnetic object. From this, also the friction for the respective contact area can be determined. Based on these principles a physically possible decay time of the rotation of the magnetic object of about 20 s for a retaining surface comprising DCL and about 6 s for glass can be calculated. Realistic decay times of about 0.5 s are possible for glass without taking further measures for decreasing the friction.

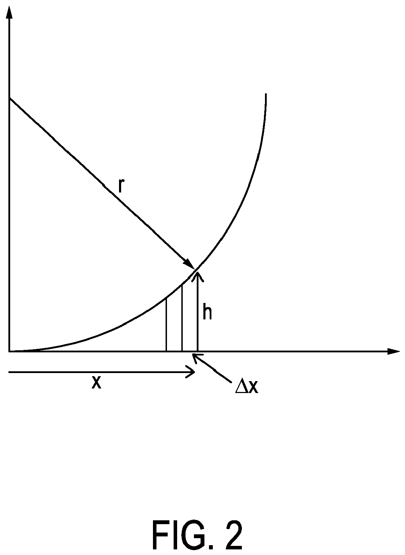

Moreover, as will be discussed with respect to Fig. 2, a lubricant oil can be provided as part of the rotary bearing 112, in particular, in the area of the contact point between the magnetic object 113 and the retaining surface, but preferably also in other regions in which a contact of the rotating magnetic object 113 and some surface might occur. In the following an approximation based on a simplified lubrication model will be provided that shows that providing such a lubricant oil has an advantageous influence on the friction between the magnetic object 113 and the retaining surface. In this simplified model it is assumed that viscosity drag acts only in columns, i.e. rings, without any lateral interaction. The magnetic object, being assumed to be spherical, is further described in this model by:

$$h = r - \sqrt{r^2 - x^2} = \frac{x^2}{2r} + O(x^4),$$

wherein h refers to the height of the magnetic object above the retaining surface at a distance x from the contact point, and $r$ to the radius of the magnetic object. The force by one ring can then be regarded as:

$$\Delta F = \frac{\eta v A}{h} = \frac{\eta \omega x A}{h} = \frac{\eta \omega x 2\pi x \Delta x}{h},$$

with $\eta$ being the viscosity, $\omega$ the rotation angular frequency, $v = \omega x$ the ring velocity and $A = 2\pi x \Delta x$ the ring surface. The torque can then be described as the force times the distance, and hence as:

$$\Delta T = \Delta F x = \frac{2\pi\omega\eta x^3}{h}\Delta x = \frac{2\pi\omega\eta x^3}{r - \sqrt{r^2 - x^2}}\Delta x \approx 4\pi\omega\eta r x \Delta x.$$

An integration of this equation gives the total torque for an oil drop with radius x:

$$T = 2\pi\omega\eta rx^2.$$

The torque provided by the magnetic forces acting on the magnetic object can be regarded as:

$$T_m = mB = \frac{1}{\mu_0}VMB = \frac{4}{3\mu_0}r^3\pi MB,$$

with m being the magnetic dipole moment, $M$ the magnetization of the sphere, and $B$ the external flux density. Therefore, the maximum possible rotation frequency where $T = T_m$, is given by:

$$\omega = \frac{2MB}{3\mu_0\eta}\frac{r^2}{x^2} = \frac{2MB}{3\mu_0\eta}\alpha^2,$$

with $\alpha = \frac{r}{x}$ the relative size of the oil droplet. Typical values for the parameters in this equation are, for instance, $\eta = 3$ mPas, $M = 1$ T, $B = 1$ mT. Based on these values a frequency of $f = 42$ $kHz$ $\alpha^2$ can be calculated. A useful lubrication oil comprises, for instance, a relative droplet size of $\alpha < 0.1$, which means that for typical sphere diameters of 0.5 mm, the lubrication does not hinder fast rotation and is in the order of damping due to the air in the device. Generally, if the magnetic object referring to a sphere rotates very fast, the lubricant oil will be expelled by centrifugal forces. However, this has the advantage that the system is automatically left with the right amount of lubrication provided that the surface wettability is chosen right. Preferably, the lubricant oil is a ferrofluid. This can lead to a distance between the magnetic sphere and the retaining surface. Due to this distance, the friction can be reduced further. Moreover, all components that comprise potential contact areas, like the magnetic object and the retaining surface, can be provided with additional layers that are configured to further reduce a friction between the components.

In the following, some exemplary embodiments will be shown and discussed in detail for a further reduction of the normal force acting on the contact point, for instance, by improving the stability of the position of the magnetic object 113 relative to the rotary bearing 112. Also other advantages might be provided by the respective embodiments.

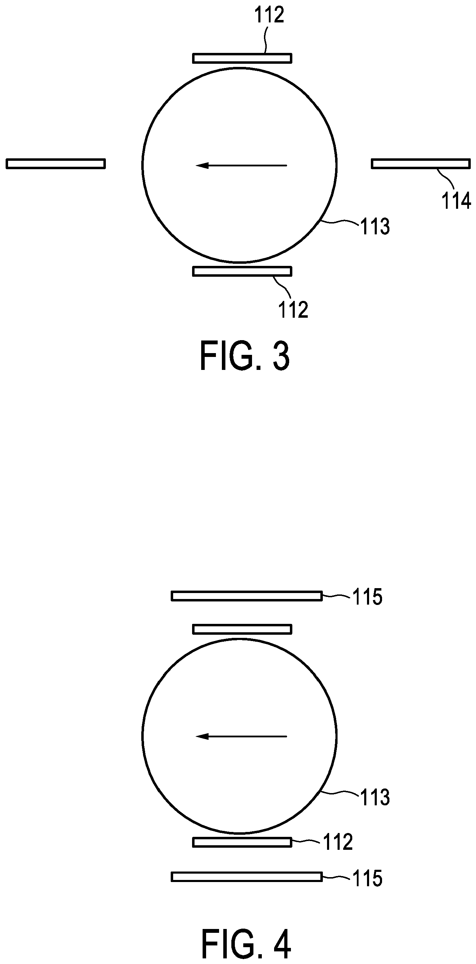

Fig. 3 shows a set-up similar to the set-up already discussed with respect to Fig. 1, wherein in this case a supplemental soft magnetic ring 114 is added as part of the rotary bearing 112. The supplemental soft magnetic ring 114 is preferably arranged between the soft magnetic discs 112 and in a plane parallel to the plane provided by the planar surfaces of the soft magnetic discs 112. Thus, the supplemental soft magnetic ring 114 is arranged around a rotational equator of the magnetic object 113. The supplemental magnetic ring 114 has a further stabilizing effect on the orientation and position of the magnetic object 113 with respect to the soft magnetic discs 112.

A further advantageous embodiment is shown in Fig. 4. In this embodiment, additional soft magnetic sheets 115 are added above and below the retaining magnetic discs 112. Preferably, the additional soft magnetic sheets 115 also have the form of soft magnetic discs. However, they can also have any other two-dimensional form in the plane parallel to the surfaces of soft magnetic discs 112. Also these additional soft magnetic sheets 115 can provide an additional stabilization of the orientation and position of the magnetic object 113.

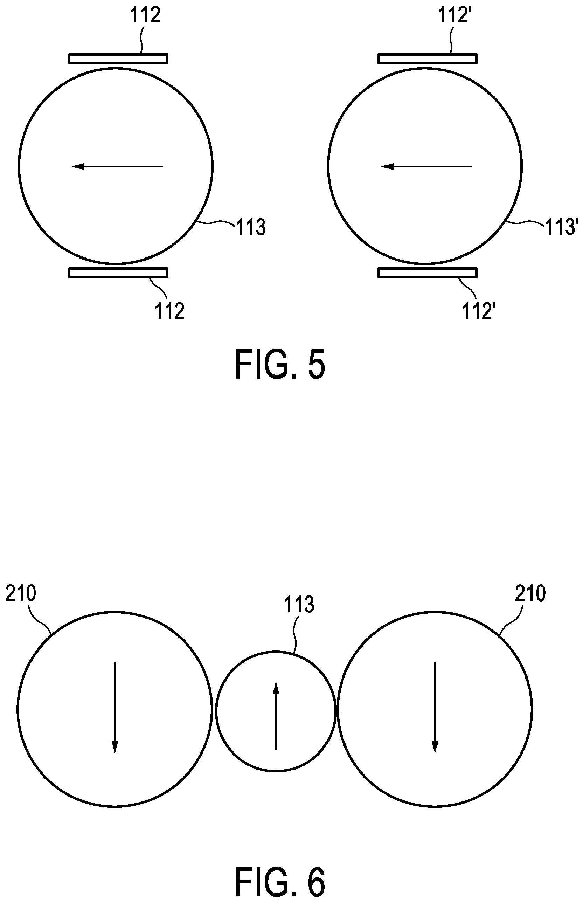

Fig. 5 shows a preferred modification that can be combined with any of the above already described embodiments. In this embodiment, the medical microdevice is provided with an additional magnetic object 113' comprising its own rotary bearing 112' that is similar to the rotary bearing 112 provided for the first magnetic object 113. In particular, in this embodiment it can be regarded that the medical microdevice is provided with an additional magneto mechanical rotator. Preferably, each of the magneto mechanical rotators is provided with its own casing structure to avoid a direct touching of the magneto mechanical rotators. However, also other structures can be provided that prevent a touching of the two magneto mechanical rotators, for instance, structures can be attached to the rotary bearings 112, 112' that allow to determine the position of the rotary bearings 112, 112' and thus also the position of the magnetic objects 113, 113'. This embodiment allows for a very good stabilization of the rotational orientation and position of the rotating magnetic objects 113, 113'. Generally, this set-up allows for a rotational oscillation of the two magnetic objects 113, 113', wherein the oscillation frequency of the rotational oscillation strongly depends on the distance between the two magnetic objects 113, 113'. Thus, this embodiment can be advantageously utilized for generating a periodically changing response magnetic field that not only allows a localization of the medical microdevice, but further allows to encode information, for instance, on physical parameters in the environment of the medical microdevice or an identification information, into the generated response magnetic field. For example, at least one of the magneto mechanical rotators can be provided with an attachment, for instance, a bellows, that allows for a change of the distance between the two magneto mechanical rotators in dependency of a change of a physical parameter in the environment of the medical microdevice. In an example, the attachment can be sensitive to a temperature and/or pressure change in the environment and react to such a change with an elongation or contraction leading to a distance change and thus an oscillation frequency change of the magneto mechanical rotators. Such a change in the frequency of the generated response magnetic field of the two magneto mechanical rotators can be easily detected, for instance, based on calibration measurements or simulations associated with a respective change in the physical parameter. Based on calibration measurements performed with a medical microdevice in an accurately defined environment, and comparisons between actual measurements of the generated response magnetic field and the measurements made during the calibration, even absolute values of physical parameters in the environment of the medical microdevice can be determined. To facilitate such measurements and to avoid the problem that the energy between the two magneto mechanical rotators is transferred in this embodiment to a "silent" oscillation mode with a zero first order magnetization change, it can be advantageous to provide the two magnetic objects 113, 113' with different sizes.

Fig. 6 shows an embodiment of the magneto mechanical rotator in which the retaining magnetic field generator comprises as two retaining permanent magnetic objects two permanent magnetic spheres 210. Also in this embodiment the retaining surface is provided by the two retaining permanent magnetic spheres 210. The magnetic object 113 in this embodiment performs a rotational oscillation between the two permanent magnetic spheres 210 providing a magnetic moment that is stable relative to the casing at least on time scales that are comparable to the rotational motion of the magnetic object 113. The advantage of this embodiment is that the normal forces and the friction at the contact point between the magnetic object 113 and the retaining surface provided by the retaining permanent spheres 210 can be further decreased leading to a further improvement of the quality factor of the magneto mechanical rotator. To further reduce the normal forces acting on the contact points between the magnetic object 113 and the two retaining permanent magnetic spheres 210, it is preferred in this embodiment to provide the two retaining permanent magnetic spheres 210 with a greater diameter than the magnetic object 113. Moreover, providing the two retaining permanent magnetic spheres 210 with a greater diameter than the magnetic object 113 has the advantage that a higher oscillation frequency compared with an embodiment with equally sized magnetic objects can be reached. Generally, a higher oscillation frequency leads to a better signal.

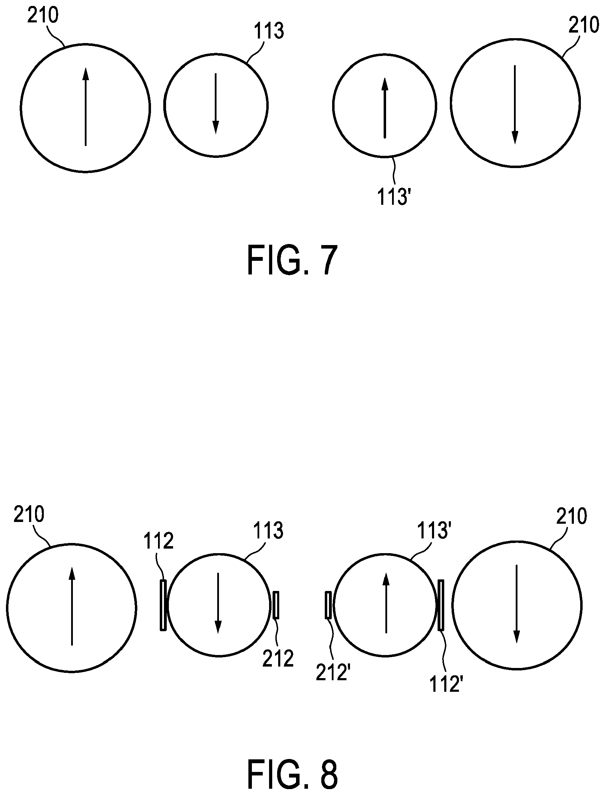

The embodiment discussed with respect to Fig. 6 can be further modified as shown in Fig. 7. In this embodiment, an additional rotating magnetic object 113' is provided between the two retaining permanent magnetic objects 210. This leads to a torque balanced set-up for a rotational oscillation of the two rotating magnetic objects, 113, 113'. Preferably, to increase the stability of this system the permanent magnetic objects 210 are provided larger than the rotating magnetic objects 113, 113', for instance, in case of spheres with larger diameters. It is further preferred that holding structures are provided to avoid the tendency of the magnetic objects 113, 113' to stick together. Such holding structures can be realized by providing a simple dividing wall between the two rotating magnetic objects, 113, 113' or by providing a bellows between the two rotating magnetic objects that still allows for the rotational oscillation and optionally for distance changes between the rotating magnetic objects 113, 113', but prevents a direct contact between the rotating magnetic objects 113, 113'. A distance variation naturally changes a magnetic interaction of this system and thus can be utilized to encode a change of a physical parameter in the environment of the medical microdevice in the generated response magnetic signal. The influence of the physical parameter can be provided, for instance, by attaching at least one of the rotating magnetic objects 113, 113' to a bellow structure that reacts with a distance change to changes of the physical parameter in the environment.

The above described embodiment can be further advantageously modified by adding as part of the retaining magnetic field generator further soft magnetic discs 212, 212' to each of the rotating magnetic objects 113, 113' as shown in Fig. 8. The additional soft magnetic discs 212, 212' can be provided according to the principles already described above, for instance, with respect to the embodiment shown in Fig. 1. Preferably, the additional soft magnetic discs 212, 212' can be provided as part of a holding structure for stabilizing the two rotating magnetic objects, 113, 113'. It is further preferred in this embodiment that the two retaining magnetic discs 212, 212' on the opposite sides of one of the rotating magnetic objects 113, 113' do not comprise the same size, i.e. do not have the same diameter. In particular, it is preferred in order to increase the stability of the set-up that the retaining soft magnetic discs 212, 212' facing each other directly have a smaller diameter than the two retaining soft magnetic discs 212, 212' between the rotating magnetic object and the retaining permanent magnetic objects 210. The additionally provided soft magnetic discs 212, 212' allow to advantageously modulate the normal forces on the contact points between the magnetic objects 113, 113' and the retaining surfaces formed here, as already described above, by the soft magnetic discs 212, 212' and at the same time further allows to reduce the size of the two retaining permanent magnetic objects 210 in relation to the magnetic objects 113, 113'. In particular, in this embodiment it can be even advantageous to provide the two retaining permanent magnetic objects 210 with a size, for instance, a diameter, smaller than the size of the rotating magnetic objects 113, 113'. Such a modification allows for a complete rotational movement of the rotating magnetic objects 113, 113'. For this case, a distance between the rotating magnetic objects 113, 113' is not encoded in the oscillation frequency of the magnetic objects 113, 113' but in the shape, i.e. harmonic spectrum, of the generated response magnetic field. This allows for an even more accurate determination of the distance and thus of possible physical parameters influencing the distance between the rotating magnetic objects 113, 113'.

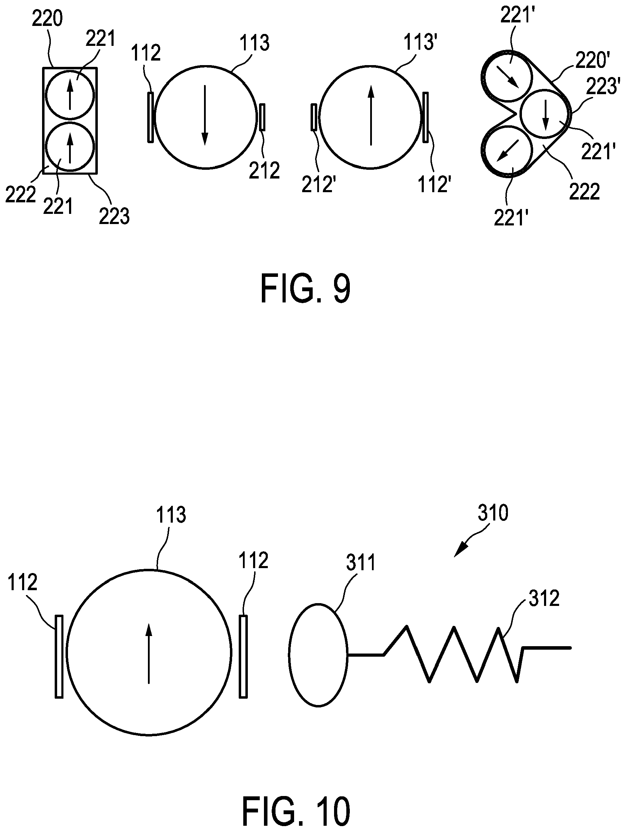

A further advantageous modification of the above described embodiment is shown in Fig. 9. This embodiment provides a torque-balanced set-up with the addition of soft magnetic disks 212, 212' and pseudo-soft magnetic permanent magnet sphere assemblies 220, 220' instead of the retaining permanent magnetic objects. The pseudo soft magnetic permanent magnet sphere assemblies 220, 220' comprise at least two permanent magnetic objects 221, 221' that are arranged such that they can move with respect to each other, in particular, can adjust the orientation of their magnetic moments with respect to each other. Other movements can be impeded by encasing the at least two permanent magnetic objects 221, 221' with respect to casing structures 223, 223' that substantially fix a positional arrangement of the least two permanent magnetic objects 221, 221' without hindering a rotational motion of the least two permanent magnetic objects 221, 221' and thus an adjustment of the magnetic moments. However, instead of an encasing structure 223, 223', also other structures can be provided that provide a fixation of the positional arrangement without impeding a rotational motion. The advantage of this assembly is that it is not destroyed if a strong magnetic field is applied and thus allows for an MRI compatibility of the medical microdevice. The advantage over providing a soft magnetic material as retaining magnetic field generator is that these assemblies 220, 220' retain a permanent magnetization. In this arrangement, it is preferred that a very high viscosity fluid 222 is provided between the permanent magnetic objects 221, 221' that serves as a lubricant, but at the same time keeps the spheres fixed on the time scale of the rotational motion of the rotating magnetic objects 113, 113', thereby avoiding energy dissipation. The simplest set-up is a two sphere assembly 220 as shown on the left, but more complicated assemblies 220' consisting of curved chains may also be utilized as shown on the right.

Fig. 10 shows a preferred embodiment of the medical microdevice comprising, in addition to the magneto mechanical rotator, a signal modulator 310. A signal modulator 310 or any of its modifications described with respect to the following figures can be combined with any of the already described embodiments of a magneto mechanical rotator. However, it is particularly preferred to provide a signal modulator 310 in embodiments of the medical microdevice comprising magneto mechanical rotators which are adapted such that the magnetic object 113 transduces the excitation field into a complete rotational motion. In embodiments with a magneto mechanical rotator which is arranged such that the external excitation field is transduced in a rotational oscillation, a signal modulator 310 can also be provided, but is less advantageous than in the embodiments with the complete rotational motion.

Generally, the signal modulator 310 can refer to any arrangement that allows to modulate the response magnetic field generated by the rotating magnetic object 113. A simple embodiment of the signal modulator 310 is shown in Fig. 10. In this embodiment, the signal modulator comprises a soft magnetic element 311 and a bellows 312 that allows to change a distance between the soft magnetic element 311 and the magneto mechanical rotator. In this arrangement, the signal modulator 310 is preferably arranged along a rotation axis of the magnetic object 113. This allows to keep lateral forces that influence an orientation of the rotating magnetic object small. However, also other arrangements of the signal modulator 310 with respect to the magnetic object 113 are generally possible, and can be advantageous for some applications. In particular, in embodiments comprising a magneto mechanical rotator with a magnetic object 113 providing a complete rotational motion, a modulation of the generated response magnetic field allows to encode information, like a physical parameter or an identity of the medical microdevice, in the generated response magnetic field. For instance, the signal modulator 310 can produce harmonics or can incorporate a resonance into the generated response magnetic field as modulation. Preferably, the signal modulator 310 comprises a magnetization change comparable to the response magnetic field change caused by the rotating magnetic object 113 in order to generate a substantial signal modulation. Preferably, the signal modulator 310 is adapted to modulate the signal generated by the rotating magnetic object 113 such that at least 1 %, more preferably 10 %, of the signal received, for instance, by the transducer, is caused by the signal modulator 310. If the signal frequency should be similar to the rotation frequency of the magnetic object 113, the signal modulator 310 preferably has a similar size as the rotating magnetic object 113. If the signal modulator 310 should generate higher harmonics in the generated response magnetic field, the signal modulator 310 on the other hand is preferably smaller than the rotating magnetic object 113.

The advantage of the use of the rotating magnetic object together with the signal modulator is two-fold. First, it allows to operate the medical microdevice in a magnetic field with lower strength. For example, within the medical microdevice, i.e. between the rotating magnetic object and the field modulator, local operation field strengths of 50 mT are easily obtained, while an external magnetic field of more than 2 mT is barely tolerable for the patient. Second, it is easier to measure a modulated signal and the measured modulated signal allows to easily single out the signal indicative of a physical parameter or an identity of the medical microdevice. In particular, it is preferred that the signal modulator is coupled to a physical parameter in the environment of the medical microdevice. The physical parameter can, for instance, be translated to a movement of the signal modulator. The simplest coupling to the signal modulator is realized by changing a distance of the signal modulator to the rotating magnetic object. However, also other couplings are possible that can even be more effective. For instance, an internal structure of the signal modulator can be mechanically changed based on the physical parameter. Especially for temperature measurements, the signal modulator may react directly to the physical quantity, for instance, by changing a value of the saturation magnetization.

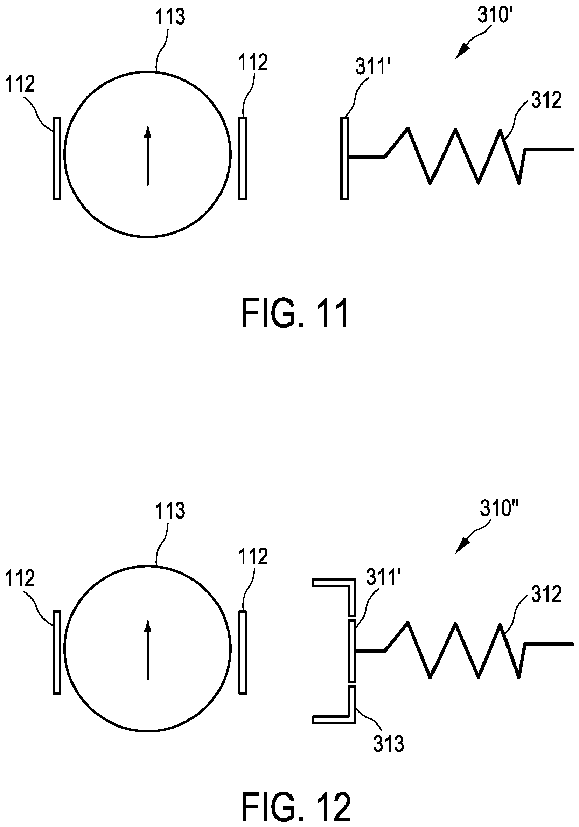

In a preferred modification of the above described embodiment the soft magnetic element 311 of the modified signal modulator 310' refers to a soft magnetic foil 311' as shown in Fig. 11. Preferably, the soft magnetic foil 311' comprises an asymmetric shape. Due to shape anisotropy the foil resists magnetization, wherein the resistance is higher in the shorter direction than in the longer direction. So if the foil is close to the rotating magnetic object 113, it is in saturation in all orientations and no harmonics are generated. If the distance increases, it is no more saturated when the magnetic sphere dipole is aligned with the short sides of the foil. Therefore, harmonics are generated in the generated response magnetic field. The exact shape of the harmonics spectrum sensitively depends on the material and the shape of the foil 311' and can be tailored to the desired application.

Fig. 12 shows a further modification of the above simple embodiment of the signal modulator 310, wherein in this further modification the signal modulator 310" comprises additionally a flux concentrator 313. The flux concentrator 313 preferably refers to a rigid structure of soft magnetic material that amplifies the response magnetic field generated by the magnetic object 113 and also the magnetic field gradient of the response magnetic field generated by the magnetic object 113. The addition of the flux concentrator 313 has the advantage that already very small movements of the signal modulator 311", i.e. very small distance changes, produce a signal change that can be measured. Thus, a signal modulator 310" comprising a flux concentrator 313 allows for a higher sensitivity for measuring changes of a physical parameter in the environment of the medical microdevice when compared with a medical microdevice comprising a signal modulator without a flux concentrator.

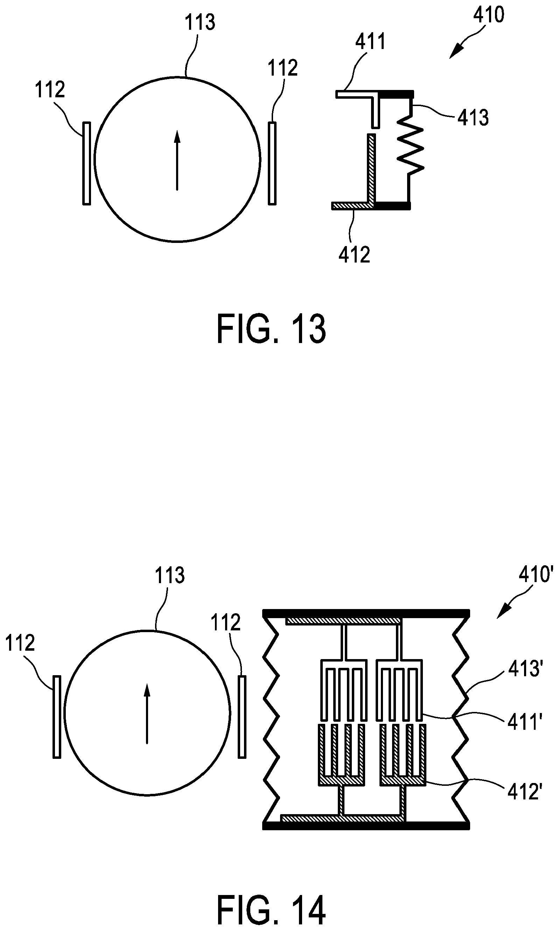

A further embodiment of the signal modulator is shown in Fig. 13, wherein the signal modulator 410 refers to a mechanical resonator that can be excited by the response magnetic field generated by the magneto mechanical rotator. In a preferred embodiment, as shown in Fig. 13, the mechanical resonator 410 comprises a) a magnetic component comprising an L-shaped permanent or soft magnetic element 412 and a flux concentrator 411, and b) a bellows 413. In the here shown exemplary embodiment, the bellows 413 connects the flux concentrator 411 comprising preferably a soft magnetic material and the magnetic element 412 to each other such that the flux concentrator 411 and the magnetic element 412 can change a distance to each other and thus an equilibrium position with respect to each other. Moreover, the restoring force provided by the bellows 413 acting against the change of the distance between the flux concentrator 411 and the magnetic element 412 leads in case of a resonant excitation of the mechanical resonator by the response magnetic field generated by the rotating magnetic object 113 to an oscillation around the equilibrium position of the magnetic element 412 and the flux concentrator 411. In particular, the mechanical resonator 410 can be arranged and adapted such that a change in the physical parameter in the environment of the medical microdevice leads to a change of the internal structure of the mechanical resonator 410, for this exemplary embodiment, to a contraction or elongation of the bellows 413 and thus a change of the equilibrium point of the magnetic element 412 and the flux concentrator 411. This change and the state of the bellows 413 then leads to a change of the resonance frequency of the mechanical resonator 410.

As a resonance frequency determination is highly desirable for an accurate parameter determination, this embodiment allows for a transformation of the signal to a higher frequency oscillation. The rotating magnetic object 113 provides a high frequency and high strength response magnetic field. By using preferably a high aspect ratio soft magnetic material, saturation can be reached and with this, high harmonics are produced. These high harmonics can then excite the mechanical resonator 410. The resonance frequency can be changed by a geometrical change of the bellows 413. The quantity to measure, i.e. the physical parameter, is in this case encoded in the phase difference between the excitation field and the harmonics in the generated response magnetic field. The rotating magnetic object 113 has here the additional benefit that both signals are fairly easy to measure simultaneously. It is possible to generate a 20th harmonics from a 100 kHz signal, and therefore the dynamic magnetic dipole of the mechanical resonator 410 can be provided small compared to the one of the rotating magnetic object 113 and still be detectable.

A more sophisticated embodiment that is also based on the principles described with respect to Fig. 13 is shown in Fig. 14. In this embodiment, the bellows 413' is provided, for instance, in form of a pressure can, i.e. a structure that reacts with a compression or elongation in one direction when subjected to changes in pressure in the environment. Within the bellow structure 413' in this embodiment the mechanical resonator 410' comprises as magnetic component at least one soft magnetic element comprising the form of an at least two pronged tuning fork. It is noted here the term "tuning fork" is only used for describing the geometrical form of the magnetic element, but not for describing its function. In the preferred embodiment shown in Fig. 14 the tuning fork comprises four prongs, however, also a two or three pronged tuning fork or a tuning fork with more than four prongs could be utilized. In this embodiment, also the flux concentrator 411' can be regarded as magnetic element and comprises the same form as the magnetic element 411', wherein the two magnetic elements, i.e. the magnetic element 412' and the flux concentrator 411', are arranged such that the prongs of the tuning forks face each other. Generally, also the principles of a mechanical resonator utilized as signal modulator in a medical microdevice as described above can be applied to this embodiment. The magnetic elements in form of a tuning fork are preferably made partially out of very thin and/or flexible material, so that a resonance frequency is low. Where the two tuning forks almost touch, they are split into a multitude of poles, which makes the attractive forces relatively large and significantly increases the resonance frequency of the whole assembly in the magnetized state. As the forces are only high if the poles are close to each other, the resonance frequency strongly depends on the distance, which is changed due to, for instance, a pressure applied to the pressure can. During the oscillation, the effective gap changes its width. If the external magnetic excitation field is sufficiently low, this changes the magnetization of the soft magnetic material and therefore the magnetic dipole moment. The tuning forks are preferably a little misaligned so that the changing field produces an exciting force. Harmonics are generated by the same soft material of the tuning forks and the right conditions for magnetization change are periodically met by the strong field change of the rotating magnetic object.

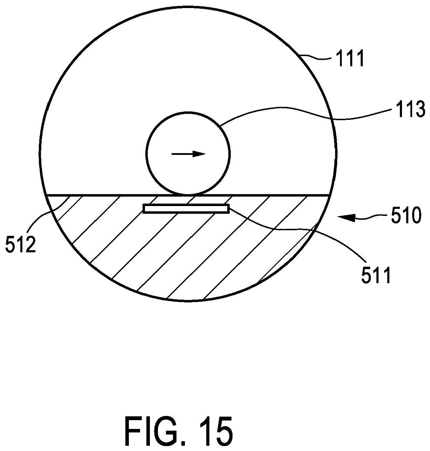

Fig. 15 shows another embodiment of a rotary bearing of a magneto mechanical rotator, wherein in this case the rotary bearing 510 does not comprise a saddle point. In this embodiment, the magneto mechanical rotator is provided with the rotary bearing 510 comprising a contact surface 512 which can comprise similar characteristics as the contact surfaces described above and which is in contact with the magnetic object 113. To hold the magnetic object 113 under the contact surface 512, the rotary bearing 510 is further provided with a permanent or soft magnetic foil 511 arranged beneath the contact surface 512. Preferably, the magnetic foil 511 is arranged parallel to the contact surface 512. The magnetic object 113 will then substantially arrange itself such that the magnetic moment of the magnetic object 113 is parallel to the contact surface 412 and the rotation axis is substantially perpendicular to the contact surface 512. Generally, although embodiments that do not comprise a retaining magnetic field generator adapted for generating a retaining magnetic field such that it is provided with a saddle point provide less stability, they can be easier produced and for some applications still be useful. To stabilize the rotating magnetic object 113 in this embodiment and to decrease a normal force on the contact surface 512, the contact surface 512 can comprise at least partly a concave shape encompassing at least a part of the magnetic object 113. In this case, the magnetic forces holding the magnetic object 113 at the contact surface 512 can be reduced, leading to a reduction of the normal force. Moreover, a lateral stability of the magnetic object 113 is increased.

Generally, the main idea of the invention refers to a magneto mechanical rotator comprising as first component a fast rotating permanent magnetic object. For this, the rotating magnetic object, possibly a sphere, is provided with some sort of rotary bearing. The rotary bearing can be realized advantageously in a plurality of ways, as already described above. One of the easiest ways is that the magnetic object is held in place on a flat contact surface by a magnetic foil. The magnetization of the magnetic foil is preferably perpendicular to the rotation axis. An oscillating or rotating external magnetic field can then spin the rotation of the magnetic object up by using, for instance, a suitable sweep from low to high frequencies, i.e. a chirped magnetic field. The maximal rotation speed of the rotating magnetic object is limited by its material strength to approximately 100 m/s at the rim. For a magnetic object realized as a 0.5 mm sphere, this approximation gives a maximal rotation speed of at least 64 kHz. To allow for an accurate detection of the signal provided by the rotating magnetic object, it is preferred that the friction is low enough that the signal can be recorded after the excitation. So, for instance, for several 10 rotations the high speed needs to be maintained. The detailed embodiments described with respect to the figures provide examples on how to achieve such a low friction and accurate detection of the signal.

Generally, a simple and efficient rotary bearing for the fast rotating magnetic object can comprise a soft magnetic material that is brought close to the rotating magnetic object. The material can then reach magnetic saturation, wherein this saturation can generate harmonics in the generated response magnetic field, in particular, if a suitable anisotropy is maintained. The simplest embodiment can refer to providing a magnetic needle symmetrical at, but perpendicular to the rotation axis of the rotating magnetic object. If close enough, the needle gets in saturation and the resulting generated response magnetic field contains harmonics. These harmonics can then be evaluated, for instance, to decode a physical parameter or for an identification of the medical microdevice. Technically it is not even necessary to reach magnetic saturation. In such a case, the medical microdevice's generated rotationally symmetric response magnetic field just gets distorted to a more elliptical magnetic field. Such distortions can also be recorded using, for example, more than one transducer for transducing the response magnetic field to an electric response signal from more than one direction. To have a high signal strength, the saturation magnetic dipole moment of the needle is preferably close to the rotating permanent magnetic object's dipole moment. This allows the maximum possible signal in the harmonics.

The generation of harmonics by introduction of a soft magnetic material also enables detection of the medical microdevice during the excitation by the excitation magnetic field. For example, during the measurement the excitation frequency band can be suppressed in the receive path by adequate filtering and only the spectrum of higher harmonics can be used for detection and localization.

With the set-up described so far, it is possible to construct an efficient marker, i.e. a medical microdevice, that can be localized very accurately. The markers can even have some unique structure encoded in the generated signal, which allows the distinction of several thousand types of medical microdevices. To convert the medical microdevice into a sensor, the harmonics spectrum can be altered by an external physical parameter. For example, to measure a temperature the soft magnetic needle is provided with a Curie temperature close to the desired measurement temperature. Then the generated harmonics will be indicative of the temperature. For measuring pressure, the easiest way is to move the needle closer to the rotating magnetic object by a bellows. Changes in distance alter the local field and hence the harmonics spectrum. However, for a higher sensitivity it is preferred to move a second soft magnetic needle relative to the first one and thus change the harmonics spectrum. These objects can be much closer to each other than to the magnetic object such that smaller movements can be detected. A further possibility is to move a hard magnetic object relative to the soft magnetic needle and thereby modulate the harmonics spectrum.

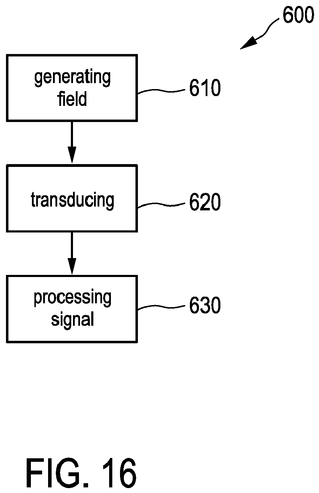

Fig. 16 shows schematically and exemplarily a method for localizing a medical microdevice and/or for determining a physical parameter in the environment of the medical microdevice. The method 600 comprises a first step 610 of generating a magnetic or electromagnetic excitation field for inducing a mechanical rotation of a magnetic object of a magneto mechanical rotator as discussed, for instance, in the embodiments above. The rotation of the magnetic object of the magneto mechanical rotator then generates a periodically changing response magnetic field. In a next step 620, the periodically changing response magnetic field is transduced into electrical response signals. In step 630 the electrical response signals are then processed, for instance, by a processor, to determine a location and/or a physical parameter and/or a change of a physical parameter in an environment of the medical microdevice based on the electrical response signals.

Although in the above embodiments the magnetic object was depicted as a permanent magnetic sphere, in other embodiments the magnetic object can also comprise other shapes, like prolate or oblate spheroidal shapes, cylindrical shapes, etc. Moreover, although in the above embodiments soft magnetic discs and/or permanent magnetic spheres were shown as rotating magnetic field generator, in other embodiment also soft magnetic cylinders, permanent magnetic discs or cylinders, permanent magnetic cuboids, differently shaped spheroids, etc., can be utilized for providing the retaining magnetic field.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Determinations like the determination of the position of the microdevice, the change of a physical parameter, or the physical parameter itself based on the generated magnetic field, etc., performed by one or several units or devices can also be performed by any other number of units or devices. These processes can be implemented as program code of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a medical microdevice for insertion into a human body, wherein the microdevice allows for measuring at least one of a localization of the microdevice in a space and/or a physical parameter in the environment of the microdevice, wherein the microdevice comprises a casing and within the casing a magneto mechanical rotator, wherein the magneto mechanical rotator comprises a magnetic object providing a permanent magnetic moment and a rotary bearing that is adapted to stabilize a rotational motion of the magnetic object, wherein the magneto mechanical rotator is adapted to transduce an external magnetic or electromagnetic excitation field into a mechanical rotation of the magnetic object relative to the rotary bearing such that a periodically changing magnetic response field is generated. The microdevice thus allows for an improved signal transmission and for a further miniaturization.

## Claims

1. A medical microdevice, wherein the microdevice allows for measuring at least one of a location of the microdevice in space and/or a physical parameter in the environment of the microdevice, comprising:

    a casing (111) and within the casing a magneto mechanical rotator, the magneto mechanical rotator comprises at least one magnetic objects (113) providing a permanent magnetic moment, and at least two retaining permanent magnetic objects (210), , the at least two magnetic objects (210) at least partially connected to the casing (111) of the medical microdevice, and further comprising
    at least one rotary bearing (112) that is adapted to stabilize a rotational motion of the at least one magnetic object (113).

2. The medical microdevice according to claim 1, wherein the at least two magnetic objects (210) are arranged on opposite sides of the magnetic object (113) such that a saddle point is provided between the at least two retaining permanent magnetic objects (210).

3. The medical microdevice according to any one of claims 1-2, wherein the at least one rotary bearing (112) comprises a retaining surface in a spatial direction perpendicular to the predefined spatial plane.

4. The medical microdevice according to claim 3, wherein the retaining surface is arranged and adapted such that minimal contact with retaining surfaces is provided, preferably only at one point.

5. The medical microdevice according to any one of claims 3-4, wherein the retaining surface comprises at least in a possible contact area a compressive strength of more than 10 GPa.

6. The medical microdevice of any one of the previous claims, further comprising a retaining magnetic field generator, the retaining magnetic field generator adapted such that a normal force applied by the magnetic object (113) on the retaining surface is lower than 1000 times, preferably lower than 100 times, than the gravitational force acting on the magnetic object (113).

7. The medical microdevice according to any one of the previous claims, wherein the at least two retaining permanent magnetic objects (210) are of a substantially cylindrical shape.

8. The medical microdevice according to any one of previous claims, wherein the at least two retaining permanent magnetic objects (210) are arranged such that the orientations of the magnetic moments of the permanent magnetic objects are fixed relative to the casing over a time scale that is comparable with the time scale of the mechanical rotation of the magnetic object.

9. The medical microdevice according to any one of the previous claims, wherein the magneto mechanical rotator (110) is adapted to transduce an external magnetic or electromagnetic excitation field into a mechanical rotation of the magnetic object (113) relative to the rotary bearing (112) such that a periodically changing magnetic response field is generated.

10. The medical microdevice according to any one of the previous claims, wherein additional magnets outside the casing (111) are deployed, the additional magnets configured for allowing a correction and/or a calibration of the magnetic field generated by the magnetic objects within the casing.

11. The medical microdevice according to any one of the previous claims, wherein at least two magnetic objects (113, 113') are used, and wherein the at least two magnetic objects (113, 113') are arranged such that they are moveable with respect to each other.

12. The medical microdevice according to claim 10, further comprising soft magnetic discs 212, 212' attached to each of the magnetic objects 113, 113' and configured to stabilize the two magnetic objects 113, 113'.

13. The medical microdevice of any one of the previous claims, wherein the magnetic objects (113, 113') are adapted to perform a rotational oscillation, rotational or oscillational movement.

14. A reading system for wirelessly reading out the medical microdevice as defined by any of the preceding claims, wherein the reading system (130) comprises:

a field generator (131) for generating a magnetic or electromagnetic excitation field for inducing a mechanical rotation of a magnetic object (113) of a magneto mechanical rotator (110) of the medical microdevice (100), wherein the rotation of the magnetic object (113) generates a periodically changing response magnetic field, a transducer (132) for sensing and transducing the response magnetic field into electrical response signals, a processor (133) for processing the electrical response signals.

15. A method for localizing a medical microdevice according to any of claims 1 to 13 and/or for determining a physical parameter in the environment of such a medical microdevice, wherein the method (600) comprises: generating (610) a magnetic or electromagnetic excitation field for inducing a mechanical rotation of a magnetic object of a magneto mechanical rotator of the microdevice, wherein the rotation of the magnetic object generates a periodically changing response magnetic field, sensing and transducing (620) the response magnetic field into electrical response signals, processing (630) the electrical response signals to determine a location and/or a physical parameter and/or a change of a physical parameter in an environment of the medical microdevice based on the electrical response signals.

16. Computer program product comprising program code for causing a reading system as defined by claim 14 to carry out the steps of the localization method and/or the measurement method as defined by claim 15, respectively, when the computer program is run on a computer controlling the reading system.

FIG. 1

FIG. 2

112

113

114

112

# FIG. 3

115

113

112

115

# FIG. 4

FIG. 5

FIG. 6

210

113

113'

210

**FIG. 7**

210

112

113

113'

210

212

212'

112'

**FIG. 8**

**FIG. 9**

**FIG. 10**

FIG. 11

FIG. 12

FIG. 13

FIG. 14

FIG. 15

FIG. 16

## REFERENCES CITED IN THE DESCRIPTION

**Patent documents cited in the description**

- WO 2019243098 A1